(19) Europäisches Patentamt
European Patent Office
Office européen des brevets

(11) Publication number: **0 416 725 A2**

(12) # EUROPEAN PATENT APPLICATION

(21) Application number: **90307566.1**

(22) Date of filing: **11.07.90**

(51) Int. Cl.5: **C12N 15/51**, C07K 15/00, //A61K39/29,G01N33/576

The microorganisms have been deposited with Fermentation Research Institut, Japan under numbers: FERM BP 2951 - 2968.

The title of the invention has been amended (Guidelines for Examination in the EPO, A-III, 7.3).

(30) Priority: **14.07.89 JP 182073/89**
**19.07.89 JP 184739/89**
**22.07.89 JP 189874/89**
**27.07.89 JP 192721/89**
**29.07.89 JP 195413/89**
**03.08.89 JP 200217/89**
**10.08.89 JP 205722/89**
**21.09.89 JP 243304/89**
**22.09.89 JP 245268/89**
**19.10.89 JP 270398/89**

(43) Date of publication of application:
**13.03.91 Bulletin 91/11**

(84) Designated Contracting States:
**AT BE CH DE DK ES FR GB GR IT LI NL SE**

(71) Applicant: **Chugai Seiyaku Kabushiki Kaisha**
**5-1, 5-chome, Ukima Kita-ku**
**Tokyo(JP)**

Applicant: **Arima, Terukatsu**
**5-13-1, Sakuragaoka**
**Kagoshima-shi, Kagoshima(JP)**

(72) Inventor: **Arima, Terukatsu**
**5-13-1, Sakuragaoka**
**Kagoshima-shi, Kagoshima(JP)**
Inventor: **Yamamoto, Osamu**
**3-30-304, Izumi-cho**
**Numazu-shi, Shizuoka(JP)**
Inventor: **Tsuchiya, Masayuki**
**5-3-301, Masago-cho**
**Numazu-shi, Shizuoka(JP)**
Inventor: **Oshima, Masanobu**
**1528-16, Nakashinden**
**Ebina-shi, Kanagawa(JP)**

(74) Representative: **Davies, Jonathan Mark et al**
**Reddie & Grose 16 Theobalds Road**
**London WC1X 8PL(GB)**

(54) **Blood-borne non-A, non-B hepatitis specific protein, DNA encoding it, and process for its production.**

(57) A DNA coding for a blood-borne non-A, non-B hepatitis specific antigenic protein and corresponding to an RNA directly isolated from a human blood or liver tissue is disclosed. This antigenic protein can be produced by using the DNA, and the antigenic protein binds to a antibody in the serum of the patient with the non-A, non-B hepatitis. Therefore, the antigenic protein is useful for the diagnostic measurement of an antibody against the non-A, non-B hepatitis specific antigen.

EP 0 416 725 A2

# BLOOD-BORNE NON-A, NON-B HEPATITIS SPECIFIC ANTIGENIC PROTEIN, A DNA CODING FOR THE PROTEIN AND A PROCESS FOR PRODUCING THE PROTEIN

## TECHNICAL FIELD

The present invention relates to a novel protein and a DNA fragment. More particularly, it relates to a blood-borne non-A, non-B hepatitis specific antigenic protein which appears specifically on the pathopoiesis of blood-borne non-A, non-B hepatitis, a DNA fragment coding for the protein, and a process for producing the protein by using the DNA fragment.

## BACKGROUND ART

The viruses of the viral hepatitis type A and type B have been discovered, the hepatitis has been successfully diagnosed by the immunological method, and vaccines for these viruses have been developed. Nevertheless, currently the entity of the hepatitis which is neither type A nor type B, i.e., non-A, non-B hepatitis, is not clear, since the pathogenic virus is present only in an extremely small quantity in organisms. Blood-borne non-A, non-B hepatitis is responsible for 90% of the occurrences of post-transfusion hepatitis, which occurs in 10 to 20% of transfused patients [Experimental Medicine vol. 7, 196 - 201 (1988)].

Research into antigenic protein associated with blood-borne non-A, non-B hepatitis has been made by various researchers using patient's blood as samples. For example, Choo et al. described in Science, 244 , 359 - 362 (1989) and European Unexamined Patent Publication No. 0318 216 Al, that a chimpanzee was infected with hepatitis by infusing the blood of a patient suffering from blood-borne non-A, non-B hepatitis, RNA was then extracted from the serum of the chimpanzee, and a fragment of a non-A, non-B hepatitis virus gene was isolated from a cDNA library prepared from the RNA. Further, it has been reported that blood infected with non-A, non-B hepatitis can be diagnosed at a probability of 60 - 70% of the blood samples infected with non-A, non-B hepatitis virus, as a result of the blood sample test carried out in Japan using an immunological diagnostic reagent based on the afore-mentioned virus gene fragment (KOSEISHO KAN-EN KENKYU RENRAKU KYOGIKAI HOKOKU 1989, 3, 13).

On the other hand, Japanese Unexamined Patent Publication No. 2576/1989 disclosed that a chimpanzee was infected with non-A, non-B hepatitis by infusing the blood of a patient suffering from the hepatitis, RNA was extracted from the liver cell of the chimpanzee, and using the extracted RNA, the gene coding the non-A, non-B hepatitis specific antigenic protein was cloned.

The former report gives insufficient information, in that the diagnostic reagent has a low diagnostic probability for blood infected with the non-A, non-B hepatitis virus, the cloned gene as described in that paper has an insufficient number of bases, compared with the assumed number of bases of the blood-borne non-A, non-B hepatitis virus gene, and there is a possibility that two or more kinds of the blood-borne non-A, non-B hepatitis viruses exist.

In the latter report, no direct evidence is shown that the cloned gene is specific to human non-A, non-B hepatitis.

In both of the investigations described above, RNA obtained through a chimpanzee was used and not RNA extracted directly from human patients suffering from the blood-borne non-A, non-B hepatitis, and thus it remains uncertain whether or not the cloned DNA truly reflects the gene for the human non-A, non-B hepatitis virus. Accordingly, new approaches must be made to meet the demand for the development of a diagnostic reagent having a higher diagnostic probability, and a vaccine.

As one such approach, one of the present inventors extracted RNA directly from the blood of human patients with non-A, non-B hepatitis, and reported successful results with a method to be described hereinafter [Experimental Medicine, Vol. 7, 196 - 201 (1989)].

## DISCLOSURE OF THE INVENTION

The present inventors conducted intensive research into the solving of the aforementioned problems, and as a result, succeeded in directly extracting RNA from the liver cell or blood of human patients with non-A, non-B hepatitis, and from this RNA cloning DNAs coding for a blood-borne non-A, non-B hepatitis specific antigenic proteins.

Therefore, the present invention provides DNAs coding for a blood-borne non-A, non-B hepatitis specific antigenic protein which appears in a human liver cell and/or blood upon the pathopoiesis of a blood-borne non-A, non-B hepatitis.

The present invention also provides a process for preparing the blood-borne non-A, non-B hepatitis specific antigenic protein, comprising culturing a host transformed by an expression vector containing the DNA.

The present invention further provides the proteins produced by the above-described process.

## BRIEF DESCRIPTION OF THE DRAWINGS

Fig. 1 shows the DNA sequence of the insert in a phage clone λHC2211;
Fig. 2 shows the DNA sequence of the insert in a phage clone λHC2246;
Fig. 3 shows the DNA sequence of the insert in a phage clone λHC512, and the corresponding amino acid sequence;
Fig. 4 shows the DNA sequence of the insert in a phage clone λHC2206, and the corresponding amino acid sequence;
Fig. 5 shows the DNA sequence of the insert in a phage clone λHC2207, and the corresponding amino acid sequence;
Fig. 6 shows the DNA sequence of the insert in a phage clone λHC2216, and the corresponding amino acid sequence;
Fig. 7 shows the DNA sequence of the insert in a phage clone λHC2220, and the corresponding amino acid sequence;
Fig. 8 shows the DNA sequence of the insert in a phage clone λHC2230, and the corresponding amino acid sequence;
Fig. 9 shows the DNA sequence of the insert in a phage clone λHC2232, and the corresponding amino acid sequence;
Fig. 10 shows the DNA sequence of the insert in a phage clone λHC2244, and the corresponding amino acid sequence;
Fig. 11 shows the DNA sequence of the insert in a phage clone λHC2248, and the corresponding amino acid sequence;
Fig. 12 shows the DNA sequence of the insert in a phage clone λHC2256, and the corresponding amino acid sequence;
Fig. 13 shows the DNA sequence of the insert in a phage clone λHC2258, and the corresponding amino acid sequence;
Fig. 14 shows the DNA sequence of the insert in a phage clone λHC2270C, and the corresponding amino acid sequence;
Fig. 15 shows the DNA sequence of the insert in a phage clone λHC2410, and the corresponding amino acid sequence;
Fig. 16 shows the DNA sequence of the insert in a phage clone λHC2533, and the corresponding amino acid sequence;
Fig. 17 shows the DNA sequence of the insert in a phage clone λHC2607, and the corresponding amino acid sequence;
Fig. 18 shows the DNA sequence of the insert in a phage clone λHC2610, and the corresponding amino acid sequence;

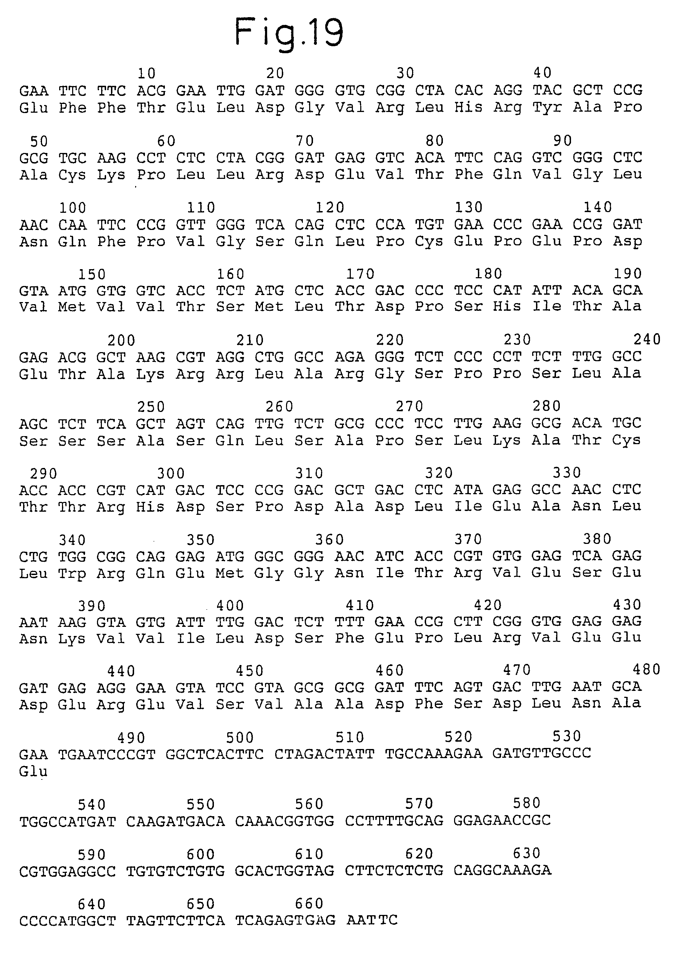

Fig. 19 shows the DNA sequence of the insert in a phage clone λHC2211 as shown in Fig. 1, and the corresponding amino acid sequence;

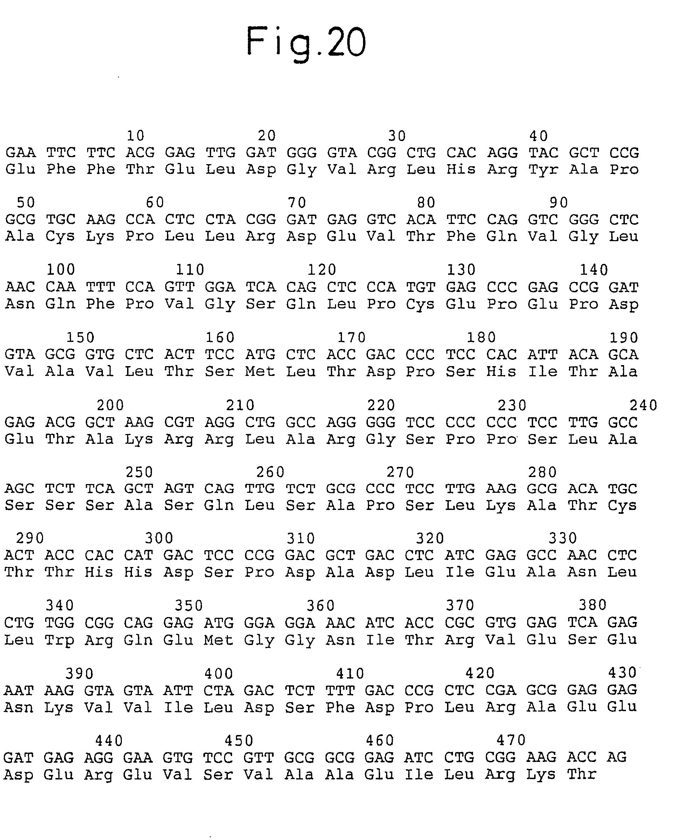

Fig. 20 shows the DNA sequence of the insert in a phage clone λHC2246 as shown in Fig. 2, and the corresponding amino acid sequence;
Fig. 21 is a schematic illustration of the construction of the expression plasmid pFETR3;
Fig. 22 shows the structure of the plasmid pTRC24 with which cDNA of the phage clone 2207 has been integrated; and
Fig. 23 shows the structure of the plasmid pFET42 with which cDNA of the phage clone 2246 has been integrated.

## BEST MODE OF CARRYING OUT THE INVENTION

The DNA coding for the blood-borne non-A, non-B hepatitis specific antigenic protein of the present invention is cloned as follows:

Namely, the total RNA is prepared by dissolving a liver tissue in a guanidinium thiocyanate solution, extracting the mixture with phenol-chloroform and precipitating the RNA with isopropanol, according to the method of Chomczynski et al. [ANALYTICAL BIOCHEMISTRY, 162 , 156 - 159 (1987)]. Alternatively, the total RNA is prepared by an ultra centrifugation of blood, and a portion of the total RNA thus obtained is purified by oligo(dT)cellulose column chromatography to isolate a poly $A^+$ RNA.

The cDNA library is obtained with the total RNA or the poly $A^+$ RNA as a template, by the random primer method described by Ebina et al., Cell, 40 , 747 - 758 (1980). The construction of the cDNA library is accomplished by using a commercially available kit (Amersham Co.; cDNA Synthesis System Plus, cDNA Cloning System-λgt 11). This kit utilizes an expression vector λgt 11, and the cDNA is integrated into the β-gal gene on the λgt 11 phage, and thus is easily expressed as the fused protein with β-galactosidase by the induction of a lactose operon promotor with isopropyl β-D-galactopyranoside (IPTG) or the like after infection of the phage with Escherichia coli .

The expression can be confirmed by the immunological screening method. This is conducted by coupling the serum of a patient suffering from the blood-borne non-A, non-B hepatitis, and an IgG fraction prepared therefrom as a 1st antibody, with an enzyme labelled 2nd antibody to develop a color [see Experimental Medicine, 6 , 958 - 964 (1988) for the principle].

The positive clones thus obtained are further screened with serums from the patients suffering from hepatitis B and sera from healthy persons, to select a clone that specifically reacts with the serum from the patients suffering from non-A, non-B hepatitis.

The DNAs thus obtained encodes all or a part of the native protein of a blood-borne non-A, non-B hepatitis specific antigen, and the DNA of the present invention is not limited to the cDNAs but includes DNAs which encode the amino acid sequence of the protein via a different codon. The DNA of the present invention is not completely identical to the cDNA, from the viewpoint of their DNA sequences, but is homologous to the extent that it can be hybridized with the cDNA under the usual conditions used for the identification of viruses. The DNAs of the present invention also include DNAs coding for a protein having the aforementioned antigenecity.

The aforementioned DNA of the present invention is useful as a material for constructing a gene system which can be used for the production of a blood-borne non-A, non-B hepatitis specific antigen, in a host such as bacteria, yeast or animal cells etc. The bacterial host includes Escherichia coli . The required antigenic protein can be produced by a conventional genetic engineering method using the cDNA which has been cloned in the present invention. For example, an expression vector such as an expression plasmid can be constructed by adding a translational initiation codon and a translational termination codon upstream and downstream of the coding region of the cDNA according to the present invention, respectively, and inserting the obtained sequence to a vector such as a plasmid, comprising has an expression control system such as a promotor, a terminator or the like, functional in a selected host.

Promoter-terminator systems which can be used, include trp promoter, lac promoter, T7 promoter, rrnB terminator, and the like in Escherichia coli , and PGK promoter, ADH 1 promoter, GAL 10 promoter, ADH terminator, and the like in a yeast such as Saccharomyces serevisiae . Also, there can be used SV40 early promoter, adenovirus major late promoter, Rous sarcoma virus LTR, SV40 poly A signal, and the like in an animal cell such as CHO, CV-1 and NIH3T3 cells. Conventional methods can be used for the construction of an expression vector, the transformation, the culture of a host, the induction of expression, and the recovery and purification of a produced protein. For example, the recovery and purification of a protein produced by E . coli can be carried out by homogenizing E . coli cells, dissolving insoluble matters containing a desired protein with 8M urea or the like, and subjecting to column chromatography on ion-exchange resin or the like. The production of the antigenic protein according to the present invention is specifically described in Example 7.

The DNA of the present invention can be used as a gene source for producing a live vaccine by integrating the DNA into a vector, such as vaccinia virus or the like.

The non-A, non-B hepatitis specific antigenic protein of the present invention is useful as a reagent for the diagnosis of non-A, non-B hepatitis when using a blood sample. For example, the lst antibody of non-A, non-B hepatitis in the serum from a patient can be detected with the antigenic protein obtained by the process of the present invention, by methods such as the Western blot method, the enzyme immunoassay method, the latex agglutination method or the radioimmunoassay method, and thus an infection with blood-borne non-A, non-B hepatitis can be diagnosed. This has been confirmed by the successful results of the experimental detection of the 1st antibody in the serum from a patient suffering from non-A, non-B hepatitis with the antigenic protein prepared in Example 7 by the Western blot method.

The present invention is explained in more detail with reference to the following Examples.

Example 1 Construction of a cDNA library

(1) Preparation of RNA

A non-cancerous tissue having a weight of ca. 1 g was obtained by an excision of liver cancer from a patient suffering from chronic blood-borne non-A, non-B hepatitis complicated with liver cancer. The total RNA was prepared from the tissue, by the method of Chomczynski et al. [ANALYTICAL CHEMISTRY, 162 , 156 - 159 (1987)].

A 10 ml portion of the solution D [4 M guanidinium thiocyanate, 25 mM sodium citrate (pH 7), 0.5% sarcosyl, 0.1 M 2-mercaptoethanol] was added to about 1 g of each of the liver tissues derived from six patients, and each mixture was homogenized and solubilized. Then, to the solubilized products were sequentially added 1 ml of 2 M sodium acetate (pH 4), 10 ml of a water saturated-phenol, and 2 ml of chloroform/isoamyl alcohol (49 : 1), and the mixture was kept on ice for 15 minutes. The aqueous phase was recovered by centrifuging the mixture at 12,000 rpm for 20 minutes at 4°C in a Sorvall ss-34 rotor, mixed with 10 ml of isopropanol and stood at -20°C for 1 hour. Then centrifugation was conducted at 12,000 rpm and 4°C for 20 minutes with a Sorvall SS-34 rotor, and the resulting precipitate was again dissolved in 3 ml of the solution D, mixed with 3 ml of isopropanol, and again stood at -20°C for 1 hour. The precipitate was recovered by centrifugation and washed with 75% ethanol, and the an amount of 0.5 mg - 1.5 mg of precipitate of the total RNA thus obtained was dissolved in distilled water.

The total RNA was prepared from blood by the following procedures to a 70 liter portion of plasma having an abnormal high ALT value and negative to HBsAg and HBV-DNA was added an equivalent amount of a diluent (50 mM Tris-HCl, 1 mM EDTA, pH 8.0), and the mixture was subjected to sucrose density gradient centrifugation at 90,000 x g to obtain a fraction having a specific gravity of 1.12 - 1.29. The fraction was dialized against to the diluent, lyophilized, and then dissolved in a solution D containing guanidinium thiocyanate. After adding poly C as a carrier, the mixture was extracted with a mixture of phenol/chloroform/isoamyl alcohol, and to the aqueous phase was added an equivalent amount of isopropanol, to precipitate nucleic acid. After this procedure was repeated once more, the nucleic acid was stored in 75% ethanol, and further, was treated with a RNase free DNase fraction which had been removed a potentially contaminated RNase in a trace amount by affinity chromatography of a commercially available RNase free DNase on agarose-5′-(4-aminophenylphosphoryl)-uridine-2′(3′)-phosphate, to remove DNA incorporated in the RNA fraction. Further, the poly C as a carrier in the remaining RNA was removed with an oligo (dG)-cellulose column and purified with NENSORB (DuPont Co.).

The poly $A^+$ RNA was prepared in the following manner. To a solution containing ca. 500 µg of the total RNA was added 20% sodium laurylsulfate to adjust to 0.5%, and the mixture was heated at 65°C for 10 minutes. After the adjustment of the mixture to 10 mM Tris-HCl (pH 8), 0.5 M NaCl and 1 mM of EDTA, the mixture was subjected to oligo(dT)cellulose (Pharmacia Co.) column chromatography. Thereafter, elution with a solution comprising 10 mM Tris-HCl (pH 7.5) and 1 mM EDTA produced a poly $A^+$ RNA, in a yield of 10 - 15 µg.

(2) Synthesis of cDNA

cDNA was synthesized with a commercially available cDNA synthesizing kit [Amersham Co.; cDNA Synthesis System Plus, (code: RPN 1256)], and to 5 µg of the RNA prepared above were added 10 µl of 5 x the first strand synthesis reaction buffer, 2.5 µl of a sodium pyrophosphate solution, 2.5 µl of human placenta ribonuclease inhibitor, 5 µl of a deoxynucleoside triphosphate mixture, 5 µl of a primer, 2 µl of [α-$^{32}$P]dCTP (Amersham PB 10205), water and 100 units of a reverse transcriptase solution, to a total volume of 50 µl. As the primer, an oligo(dT)primer was used for RNA Nos. 4 and 5 as template, and a random hexanucleotide primer was used for other RNAs. After incubation at 42°C for 40 minutes, to the first strand cDNA synthesis reaction mixture were added 93.5 µl of the second strand synthesis reaction buffer, 4 units of E . coli ribonuclease H, 115 units of E . coli DNA polymerase I and water to a total volume of 250 µl. After reaction at 12°C for 60 minutes and at 22°C for 60 minutes, the reaction mixture was incubated at 70°C for 10 minutes. Then, after adding 10 units of T4 DNA polymerase and incubating 37°C for 10 minutes, 10 µl of 0.25 M EDTA (pH 8.0) was added. The reaction mixture was extracted with phenol/chloroform, and after adding a 4 M ammonium acetate solution in an equivalent amount, subjected to ethanol precipitation. The cDNAs shown in Table 1 were obtained by repeating the above-described procedures. Note, the RNA sample Nos. 4, 5, 22, 24 and 25 are derived from liver cells and No. 26 is

derived from blood.

Table 1

| RNA Sample | Amount of RNA (μg) | Double Strand cDNA (ng) |
|---|---|---|
| No. 4 (poly $A^+$ RNA) | 15 | 1260 |
| 5 (poly $A^+$ RNA) | 10.5 | 4500 |
| 22 (total RNA) | 8 | 260 |
| 24 (total RNA) | 20 | 1400 |
| 25 (total RNA) | 22 | 260 |
| 26 (total RNA) | 5 | 5200 |

(3) Construction of cDNA library

The cDNA library was constructed using a cDNA Cloning System-λgt 11 (Amersham code RPN 1280), and to the cDNA obtained above (ca. 1 μg) were added 4 μl of the M buffer, 2 μl of 1 x SAM solution, water, and 20 units of EcoR I methylase, to a total volume of 20 μl. After reaction at 37° C for 60 minutes, the reaction mixture was heated at 70° C for 10 minutes and then 3 μl of L buffer, 2 μl of a EcoR I linker, water, and 5 units of T4 DNA ligase were added, to a total volume of 30 μl, and the mixture allowed to react at 15° C overnight. After terminating the reaction by heating at 70° C for 10 minutes, 10 μl of the E buffer, water, and 100 units of EcoR I were added to a total volume of 100 μl, and the mixture allowed to react at 37° C for 5 hours. After terminating the reaction by heating at 70° C for 10 minutes, free linkers were removed with a column equipped in the cDNA cloning system, to yield the EcoR I linker coupled cDNAs in the amounts shown in Table 2.

Table 2

| RNA Sample | EcoR I linker coupled cDNA (ng) |
|---|---|
| No. 4 (poly $A^+$ RNA) | 460 |
| 5 (poly $A^+$ RNA) | 1470 |
| 22 (total RNA) | 27 |
| 24 (total RNA) | 100 |
| 25 (total RNA) | 24 |
| 26 (total RNA) | 2200 |

To 10 - 100 ng of the cDNA were added 1 μg of the λgt 11 arm, 1 μg of the L buffer, water, and 2.5 units of T4 DNA ligase, to a total volume of 10 μl, and the mixture allowed to react at 15° C overnight. To this reaction mixture were added 10 μl of Extract A and 15 μl of Extract B, and the mixture was kept at 20° C for 2 hours to carry out in vitro packaging. Then, to the mixture were added 0.5 ml of SM buffer (50 mM Tris-HCl, pH 7.5, 100 mM NaCl, 10 mM $MgSO_4$ , 0.01% gelatin), and several drops of chloroform, to obtain a phage solution. The phage solution was titrated, and the results shown in Table 3 below were obtained as the cDNA libraries.

Table 3

| RNA Sample | Recombinant Phage Number (PFU) | Recombination Rate (%) |
|---|---|---|
| No. 4 (poly $A^+$ RNA) | $1.2 \times 10^6$ | 75 |
| 5 (poly $A^+$ RNA) | $3.2 \times 10^6$ | 82 |
| 22 (total RNA) | $3.0 \times 10^5$ | 48 |
| 24 (total RNA) | $1.3 \times 10^6$ | 48 |
| 25 (total RNA) | $2.0 \times 10^5$ | 53 |
| 26 (total RNA) | $5.1 \times 10^7$ | 89 |

Recombination rate = Recombinant phage number/total phage number x 100

Example 2 Preparation of a first antibody for the screening of the cDNA libraries

E. coli Y1090 strain was incubated with shaking in an L-broth (10 g/l bacto-trypton, 5 g/l bacto-yeast extract and 10 g/l NaCl) containing 0.4% maltose and 50 µg/ml ampicillin. Then, one ml portion of the culture was added to 50 ml of the L-broth containing 0.4% maltose and ampicillin (50 µg/ml), and the mixture was incubated with shaking at 37°C until $OD_{600}$ reached 0.5 ($2.5 \times 10^8$ cells/ml). E coli was collected by centrifugation and suspended in 5 ml of Phosphate buffered-saline (PBS). A 6 µl of 0.5 M EDTA (pH 8) and lysozyme (final concentration: 0.1 mg/ml) were added, and the mixture was kept n ice for 30 minutes. E. coli lysate was obtained by repeating freezing and thawing three times.

The 1st antibody was obtained by the procedure described below. Sera of 5 convalescents from acute non-A, non-B hepatitis and sera of 5 patients suffering from chronic non-A, non-B hepatitis were used together as the serum from patients with blood-borne non-A, non-B hepatitis. A 10 ml portion of the serum was treated in 33% saturated ammonium sulfate to give a precipitate containing immunoglobulin, the precipitate was suspended in 10 ml of TBS [10 mM Tris-HCl (pH 7.5), 150 mM NaCl], 10 ml of the aforementioned E. coli lysate was added, and the mixture was shaken at 4°C overnight or at 37°C for 1 hour. After removing the precipitate by centrifugation at 3,000 rpm for 10 minutes (HITACHI 05PR-22), 180 ml of TBS containing 1% gelatin was added, and the mixture was filtered with MILLEX-HA (0.45 µm, Millipore Co.) to give a 1st antibody.

For the serum from healthy people and the serum from a patient suffering from hepatitis B, 1 ml of the E. coli lysate was added to 1 ml of each serum, and the mixture was shaken at 4°C overnight. After removing the precipitate by centrifugation at 3,000 rpm for 10 minutes (HITACHI 05PR-22), 18 ml of TBS containing 1% gelatin was added, and the mixture was filtered with MILLEX-HA (0.45 µm, Millipore Co.) to give a 1st antibody.

Example 3 Screening of cDNA libraries

After the E. coli Y1090 strain was cultured in 10 ml of L-broth containing 0.4% maltose and ampicillin (50 µg/ml) at 37°C overnight, collected by centrifugation and suspended in 4 ml of 10 mM $MgSO_4$ to give a cell for plating. The phage solution was diluted with an SM buffer to give a concentration of ca. 6,000 PFU/100 µl and 200 µl of the cell for plating and 100 µl of the phage solution were combined and maintained at 37°C for 15 - 20 minutes. The solution was added to 10 ml of L-top agar [L-broth containing agarose (7 g/l)] and poured into a plate (EIKEN CHEMICAL CO., LTD.: No. 2 type square schale) containing L-agar (L-broth containing 15 g/l bacto-agar). After culturing at 43°C for 3 - 4 hours, a nitrocellulose filter (Schleicher & Schnell, BA85) impregnated with 10 mM IPTG (isopropyl β-D-thiogalactopyranoside) and air-dried was layered on the plate, and culturing was continued further at 37°C for 3 - 4 hours. The filter was removed and washed with TBS (10 mM Tris-HCl, pH 7.5, 150 mM NaCl) and shaken in TBS containing 5% skimmed milk (Snow Brand Milk Products Co., Ltd.) at room temperature for 1 - 2 hours. The filter was washed by shaking in TBS for 1 - 2 minutes. After repeating these procedures, the filter was dipped in the aforementioned 1st antibody, and the reaction was conducted at 4°C overnight. After washing the filter five times in TBST (TBS containing 0.05% Tween 20) for 5 minutes, it was dipped into a peroxidase conjugated anti-human IgG (goat) (Cappel Co.; code 3201-0081) solution (500-fold dilution with TBS containing 1% gelatin), to conduct the reaction at room temperature for 1.5 hours. The filter was then washed with TBST in the same manner as described above, and was reacted with a HRP-color solution [120 mg HRP-color (Bio-RAD Co.) in 40 ml of methanol, 200 ml of TBS and 120 µl of hydrogen peroxide], and the colored clone was judged positive. As a result, the positive clones as shown in Table 4 were obtained.

Table 4

| RNA Sample | Treated clone Number | Positive clone Number |
|---|---|---|
| No. 4 | 340,000 | 5 |
| 5 | 270,000 | 8 |
| 22 | 150,000 | 45 |
| 24 | 300,000 | 14 |
| 25 | 200,000 | 18 |
| 26 | 260,000 | 9 |

Single plaque isolation was conducted for the positive clones thus obtained, and their reactivities with the serum derived from healthy subjected and the serum derived from the patient suffering from hepatitis B was examined.

As a result of the reaction with the 1st antibodies derived from 5 normal subjects and 5 patients with hepatitis B in the same manner as described above, 61 clones were obtained which reacted specifically with the serum derived from patients with blood-borne non-A, non-B hepatitis. These clones (phages) are shown in Table 5.

In this connection, E. coli Y1090 strains containing these clones have been deposited with the Agency of Industrial Science and Technology, Fermentation Research Institute, Japan (address: 1-3, Higashi 1-Chome, Tsukuba, Ibaragi, Japan), and some of these strains were transferred to international deposit based on the Budapest Treaty, on June 14, 1990.

Table 5

| Phage Clone No. | National Deposit No. (FERM P.) | International Deposit No. (FERM BP.) | Deposit Date |
|---|---|---|---|
| λHC 432 | 10897 | | 1987 7 26 |
| λHC 436 | 10898 | | 1989 7 26 |
| λHC 512 | 10841 | 2951 | 1989 7 13 |
| λHC 522 | 10842 | | 1989 7 13 |
| λHC 524 | 10843 | | 1989 7 13 |
| λHC 526 | 10844 | | 1989 7 13 |
| λHC 2206 | 10845 | 2952 | 1989 7 13 |
| λHC 2207 | 10846 | 2953 | 1989 7 13 |
| λHC 2211 | 10876 | 2956 | 1989 7 21 |
| λHC 2216 | 10877 | 2957 | 1989 7 21 |
| λHC 2217 | 10852 | | 1989 7 18 |
| λHC 2220 | 10853 | 2954 | 1989 7 18 |
| λHC 2225 | 10854 | | 1989 7 18 |
| λHC 2230 | 10916 | 2966 | 1989 8 2 |
| λHC 2232 | 10930 | 2968 | 1989 8 9 |
| λHC 2239 | 10931 | | 1989 8 9 |
| λHC 2240 | 10855 | | 1989 7 18 |
| λHC 2241 | 10856 | | 1989 7 18 |
| λHC 2242 | 10857 | | 1989 7 18 |
| λHC 2243 | 10878 | | 1989 7 21 |
| λHC 2244 | 10879 | 2958 | 1989 7 21 |
| λHC 2246 | 10858 | 2955 | 1989 7 18 |

Table 5 (continued)

| Phage Clone No. | National Deposit No. (FERM P.) | International Deposit No. (FERM BP.) | Deposit Date |
|---|---|---|---|
| λHC 2248 | 10880 | 2959 | 1989 7 21 |
| λHC 2249 | 10917 | | 1989 8 2 |
| λHC 2250 | 10904 | | 1989 7 28 |
| λHC 2252 | 10881 | | 1989 7 21 |
| λHC 2255 | 10889 | | 1989 7 26 |
| λHC 2256 | 10890 | 2960 | 1989 7 26 |
| λHC 2558 | 10891 | 2961 | 1989 7 26 |
| λHC 2259 | 10892 | | 1989 7 26 |
| λHC 2263 | 10893 | | 1989 7 26 |
| λHC 2264 | 10932 | | 1989 8 9 |
| λHC 2265 | 10933 | | 1989 8 9 |
| λHC 2268 | 10894 | | 1989 7 26 |
| λHC 2270 | 10895 | 2962 | 1989 7 26 |
| λHC 2271 | 10896 | | 1989 7 26 |
| λHC 2404C | 10899 | | 1989 7 26 |
| λHC 2405B | 10900 | | 1989 7 26 |
| λHC 2410A | 10905 | | 1989 7 28 |
| λHC 2410C | 10918 | 2967 | 1989 8 2 |
| λHC 2410D | 10934 | | 1989 8 9 |
| λHC 2413 | 10919 | | 1989 8 2 |
| λHC 2414A | 10906 | | 1989 7 28 |
| λHC 2424A | 10911 | | 1989 7 28 |

Table 5 (continued)

| Phage Clone No. | National Deposit No. (FERM P.) | International Deposit No. (FERM BP.) | Deposit Date |
|---|---|---|---|
| λHC 2501 | 10920 | | 1989 8 2 |
| λHC 2502 | 10847 | | 1989 7 13 |
| λHC 2505 | 10921 | | 1989 8 2 |
| λHC 2507 | 10935 | | 1989 8 9 |
| λHC 2508 | 10859 | | 1989 7 18 |
| λHC 2509 | 10936 | | 1989 8 9 |
| λHC 2512 | 10882 | | 1989 7 21 |
| λHC 2514 | 10860 | | 1989 7 18 |
| λHC 2516 | 10861 | | 1989 7 18 |
| λHC 2533 | 10907 | 2963 | 1989 7 28 |
| λHC 2534 | 10937 | | 1989 8 9 |
| λHC 2535 | 10883 | | 1989 7 21 |
| λHC 2602 | 10908 | | 1989 7 28 |
| λHC 2603B | 10922 | | 1989 8 2 |
| λHC 2607 | 10909 | 2964 | 1989 7 28 |
| λHC 2608 | 10923 | | 1989 8 2 |
| λHC 2610 | 10910 | 2965 | 1989 7 28 |

Example 4 DNA sequence of the cDNA inserted into the phage clone λHC2211

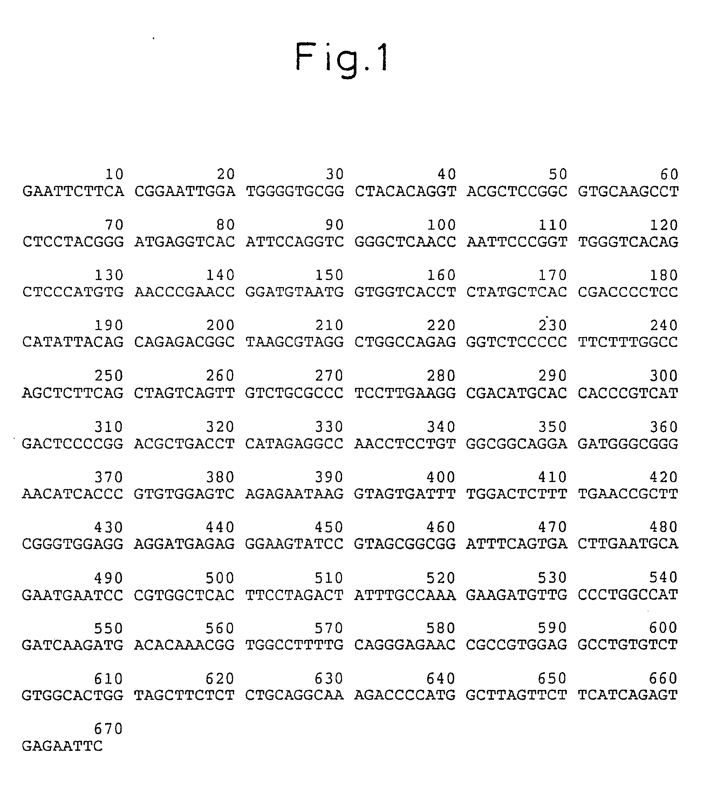

The recombinant phage λHC2211 was proliferated in the E. coli Y1090 strain, the phage was purified by a conventional method (Experimental Medicine, Vol. 5, 994 - 998, 1987), and the phage DNA was prepared by a treatment with sodium lauryl sulfate and phenol. An amount of ca. 100 μg of the phage DNA was treated with EcoR I, and ca. 0.6 μg of a DNA fragment having ca. 700 base pairs (bp) as a cDNA was recovered by 1% low melting agarose (Biolad Co.) gel electrophoresis. This DNA fragment was integrated at the EcoR I site in a phage vector M13mp18 (TARARA SHUZO), and further inserted at the EcoR I site in a plasmid pUC19 (TAKARA SHUZO), to give pMC26. Next, an EcoR I-ended cDNA fragment was prepared from the pMC26, treated with Bal I, and inserted to the Hinc II site and the Hinc II-EcoR I site of M13mp18. Also, the EcoR I-ended cDNA fragment was treated with Sau3A I and then inserted to the BamH I-EcoR I site of M13mp18. The DNA sequence of the cDNA was determined by the dideoxy method (J. Messing, Methods in Enzymology, 101 , 20 - 78, 1983) using a recombinant phage containing the M13mp18 thus obtained as a vector. The DNA sequence is shown in Fig. 1.

The blood-borne non-A, non-B virus is believed from its property to be a flavivirus [Qui-Lim Choo et al., Science, 244 , 359 (1989)]. A protein derived from the flavivirus is synthesized as a precursor polyprotein from a serial long open reading frame [E.G. Westaway, Advances in virus research, 33 , 45 (1987)]. On the other hand, the synthesis of the protein, which will be presumably synthesized from the 5′-end of the DNA sequence shown in Fig. 1, is terminated on the way of the cDNA by the presence of a terminating codon. Considering cDNA inserted to λHC2211 to be derived from the blood-borne non-A, non-B hepatitis virus which is a flavivirus, it can be assumed as an inevitable possibility in the construction of the cDNA library that two DNA fragments have been integrated at the same time. It can be also considered that the region specific to non-A, non-B hepatitis in λHC2211 may be at least a part of a protein portion in front of the terminating codon.

Example 5 DNA sequence of the cDNA inserted into the phage clone λHC2246

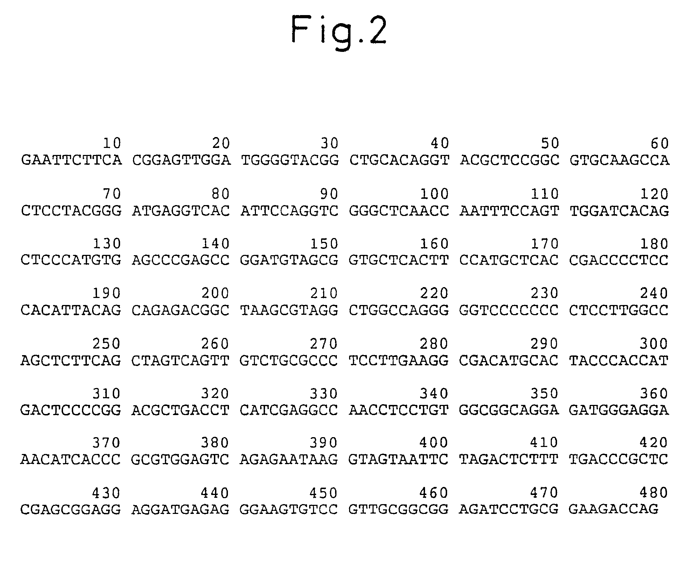

The phage DNA was prepared from the recombinant phage λHC2246 in the same manner as in Example 4. Approximately 10 μg of the phage DNA was treated with Kpn I and Sac I, and blunt-ended with a Klenow fragment of E. coli DNA polymerase I. About 0.3 μg of a DNA fragment having ca. 2.5 kilo base pairs (kb) was recovered by 1% low melting agarose (Biolad Co.) gel electrophoresis, and integrated at the Sma I site in a phage vector M13mp18 (TAKARA SHUZO). This DNA fragment was also inserted into the Sma I site of a plasmid pUC18 (TAKARA SHUZO) to give pMC42. Next, about 20 μg of pMC42 was treated with EcoR I and Pvu II, and about 2 μg of the DNA fragment having ca. 0.5 kb and containing the cDNA was recovered by 1% low melting agarose (Biolad Co.) gel electrophoresis. The EcoR I-Pvu II DNA fragment was treated with Bal I, and inserted into the EcoR I-Hinc II site and the Hinc II site of M13mp18, respectively. Using the recombinant phage on M13mp18 vector, the DNA sequence of the cDNA was determined by the dideoxy method with a Lambda gt11 primer (New England Biolad Co.) or an M13 primer (TOYO BOSEKI) (J. Messing, Methods in Enzymology, 101 , 20 - 78, 1983). The DNA sequence is shown in Fig. 2.

Example 6 DNA sequences of cDNA inserted into phage λHC512, λHC2206, λHC2207, λHC2216, λHC2220, λHC2230, λHC2232, λHC2244, λHC2248, λHC2256, λHC2258, λHC2270, λHC2410c, λHC2533, λHC2607 and λHC2610

The phage DNAs were prepared in the same manner as in Example 4 from the recombinant phages λHC512, λHC2206, λHC2207, λHC2216, λHC2220, λHC2230, λHC2232, λHC2244, λHC2248, λHC2256, λHC2258, λHC2270, λHC2410c, λHC2533, λHC2607 and λHC2610.

The EcoR I fragments containing the cDNA of λHC2206 and λHC2232 were prepared in the same manner as Example 4, and inserted into the EcoR I site of M13mp18 and pUC19, respectively. On the other hand, the Kpn I-Sac I fragments containing cDNA of λHC512, λHC2207, λHC2216, λHC2220, λHC2230, λHC2244, λHC2248, λHC2256, λHC2258, λHC2270, λHC2410c, λHC2533, λHC2607 and λHC2610 were prepared in the same manner as in Example 5, and the DNA fragments from λHC2230, λHC2256, λHC2270, λHC2410c, λHC2533, λHC2607 and λHC2610 were inserted into the Sma I site of pUC18, the DNA fragments from λHC512, λHC2207, λHC2216, λHC2220 and λHC2244 were inserted to the Hinc II site of pUC19, and the DNA fragments from λHC2248 and λHC2258 were inserted to the Kpn I-Sac I site of pUC19, respectively. Further, the Hinc II-Cla I fragment containing cDNA was recovered from the plasmid to which the DNA fragment of λHC2244 had been inserted, and inserted to the Hinc II-Cla I site of M13mp18. Using the obtained plasmids and phages, the DNA sequences of the cDNAs were determined by the dideoxy method with a Lambda gt11 primer (New England Biolabs Co.) or an M13 primer (TOYO BOSEKI) (J. Messing, Methods in Enzymology, 101 , 20 - 78, 1983). Further, oligonucleotides having the corresponding sequence to a part of the DNA sequence of the obtained cDNA were synthesized by an automatic DNA synthesizer (380A/APPLIED BIOSYSTEMS CO.). The DNA sequences of the cDNAs were also determined by the dideoxy method using the synthetic oligonucleotides as a primer.

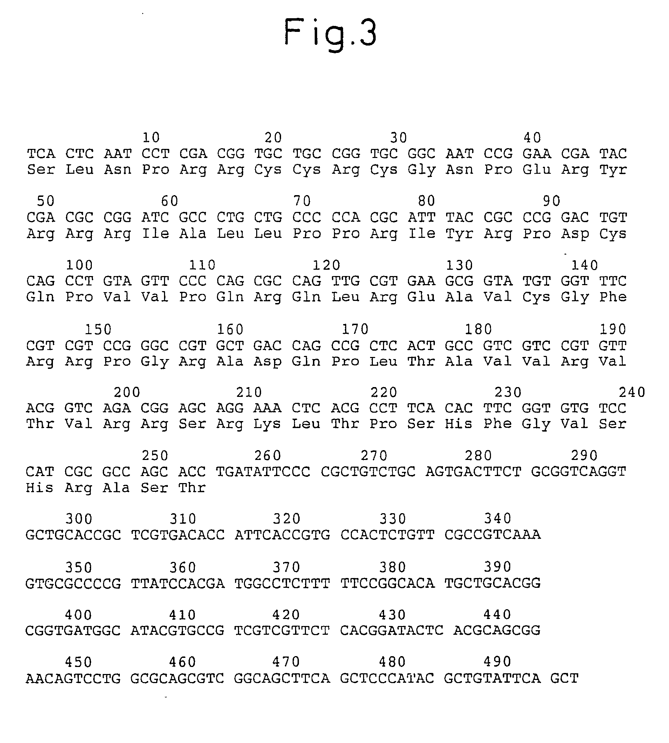

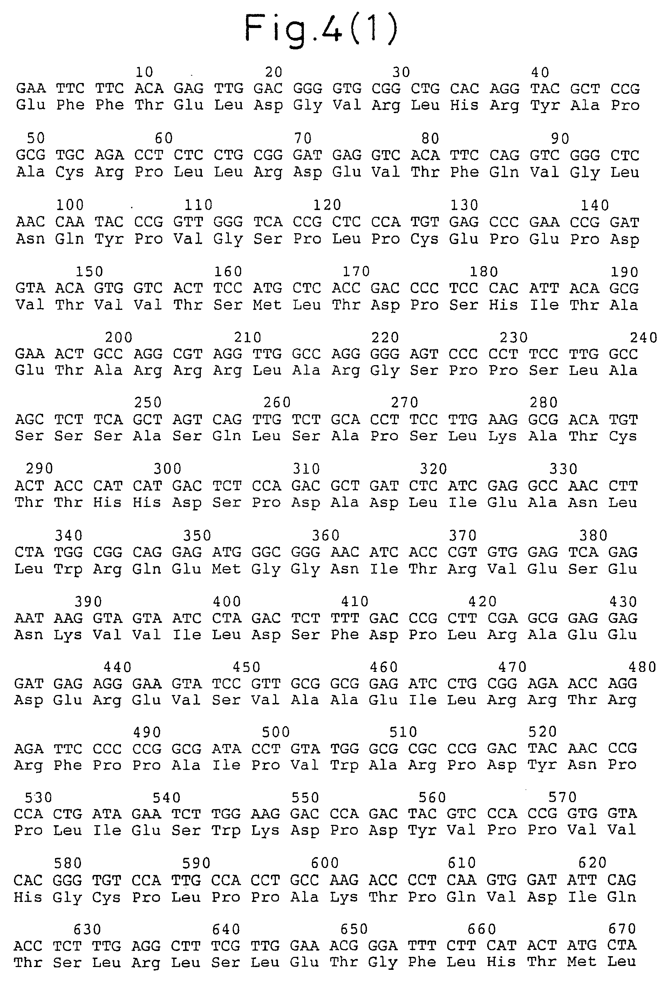

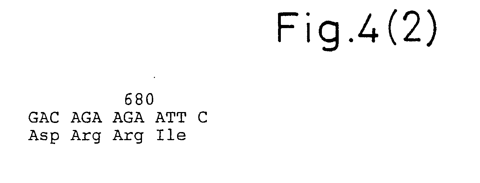

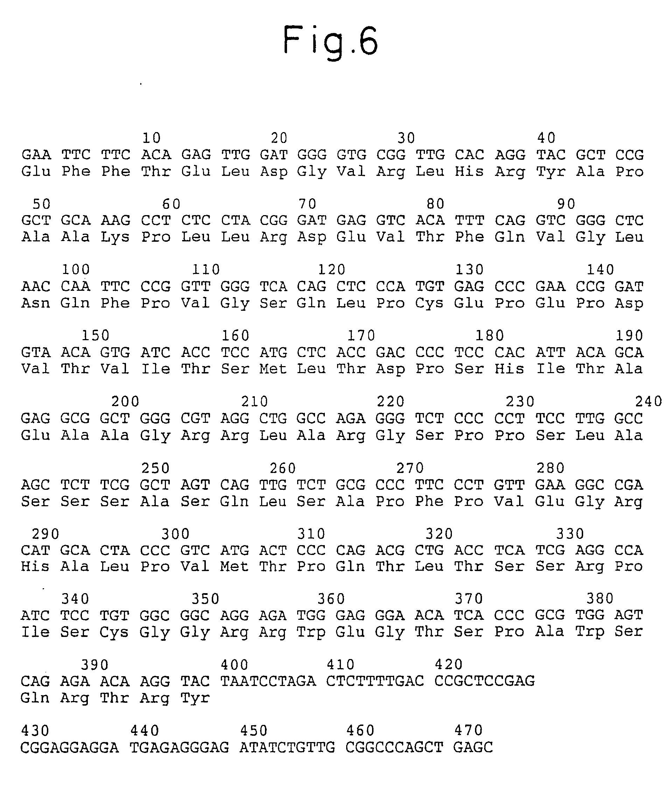

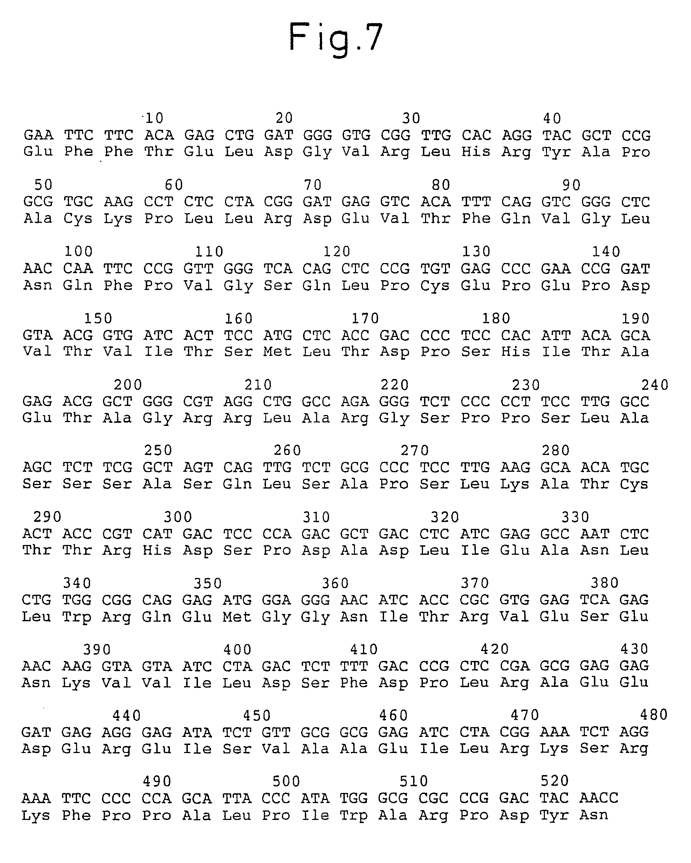

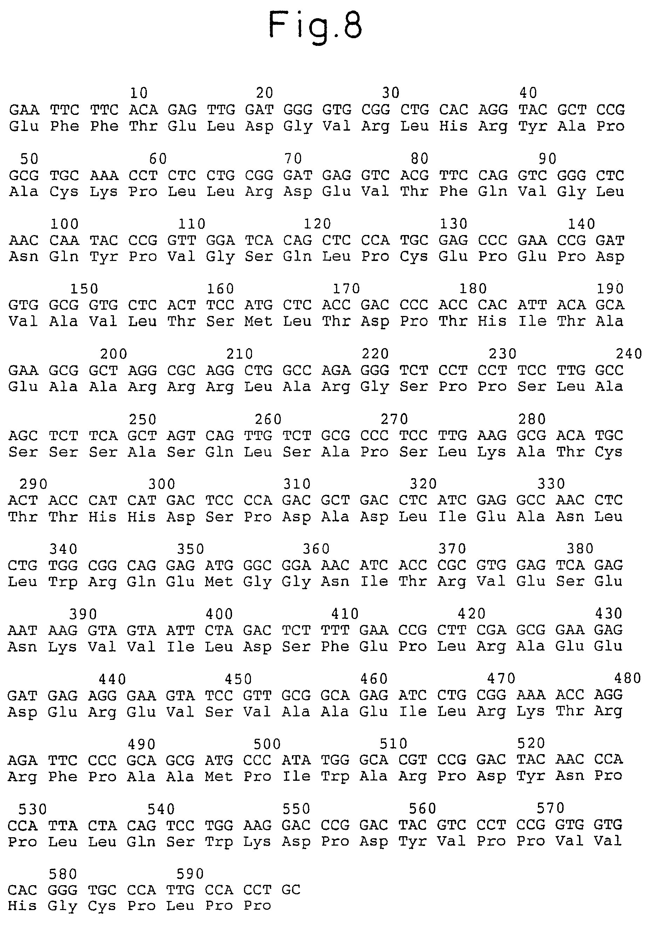

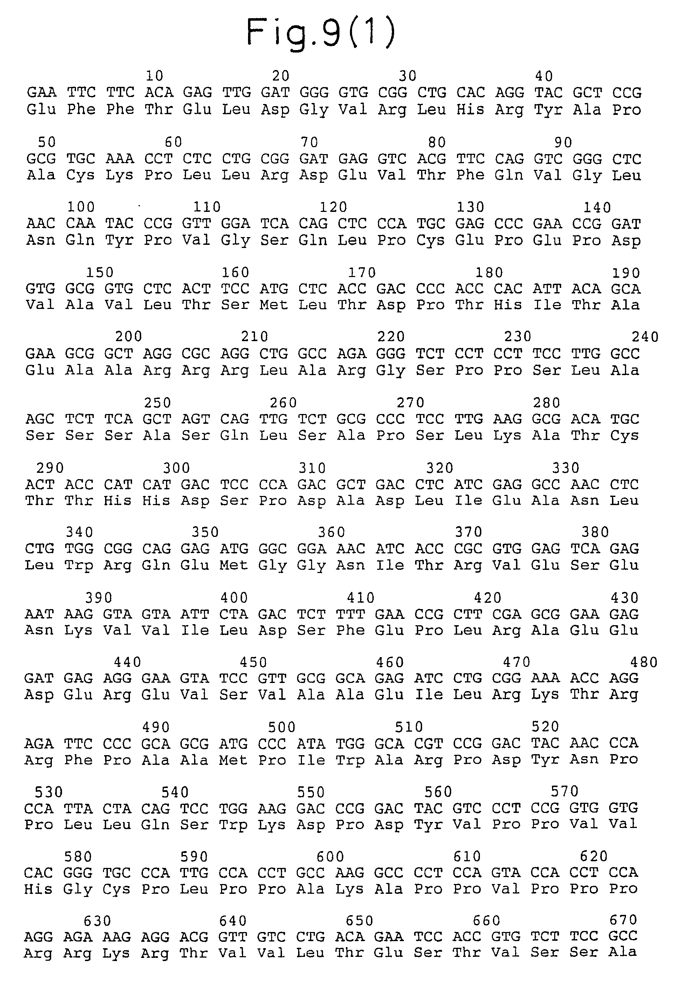

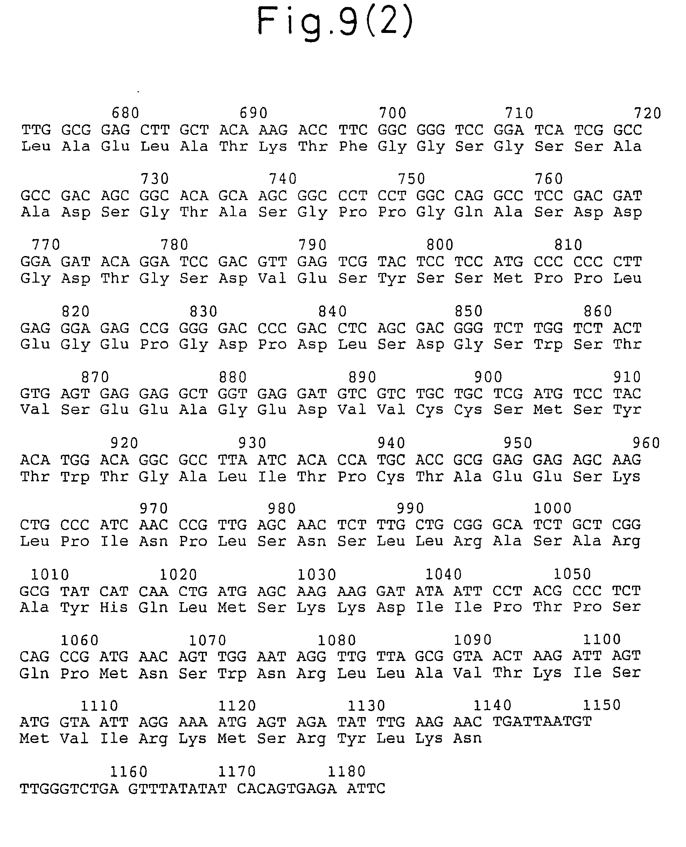

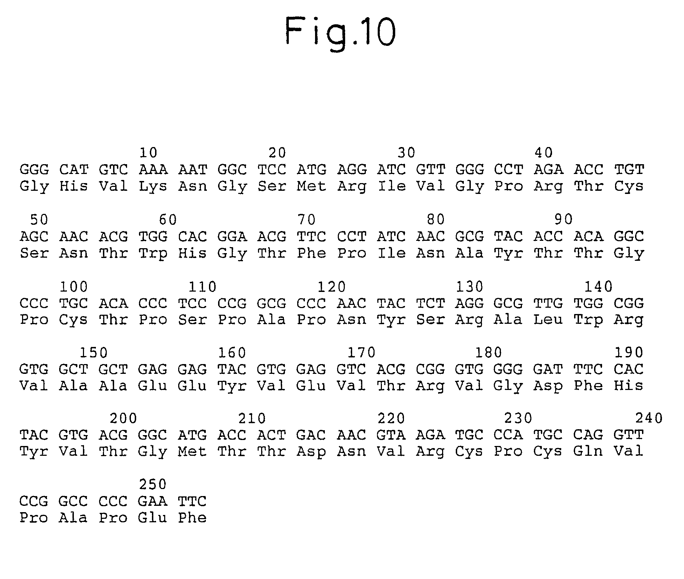

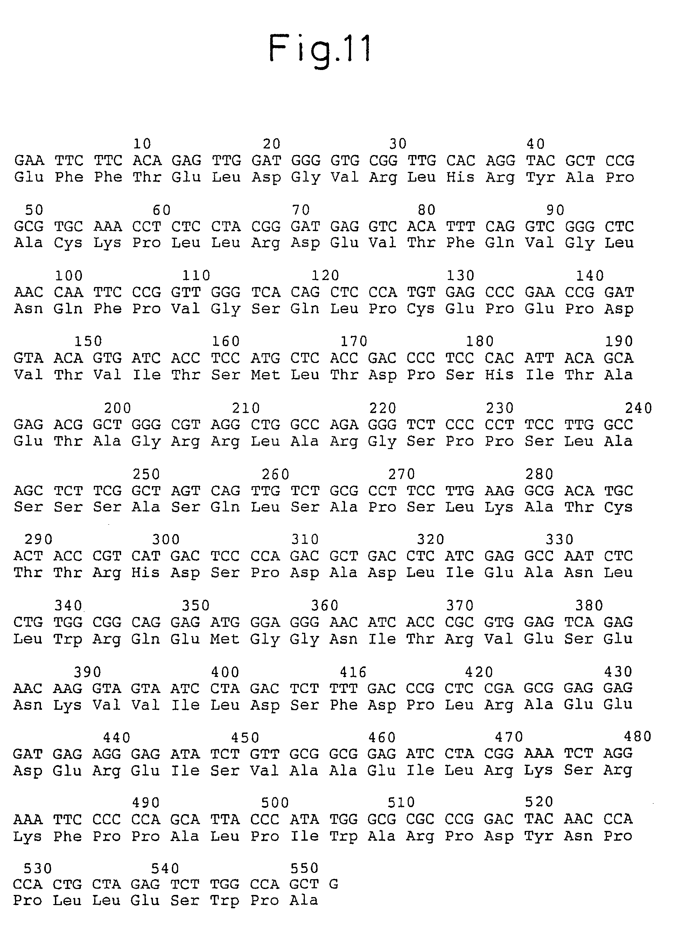

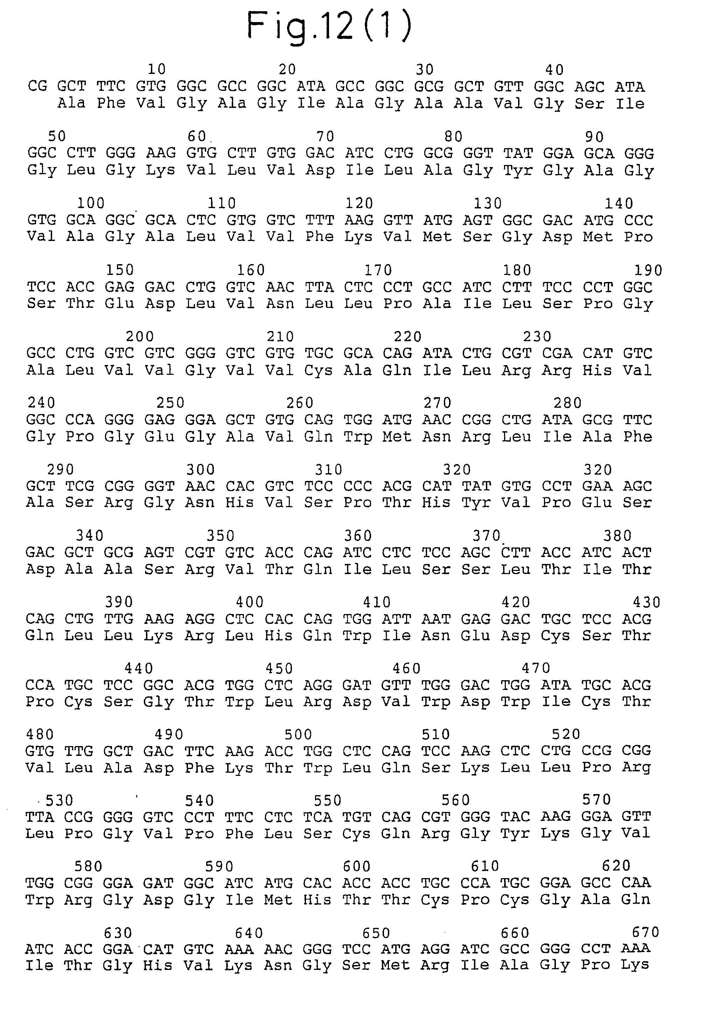

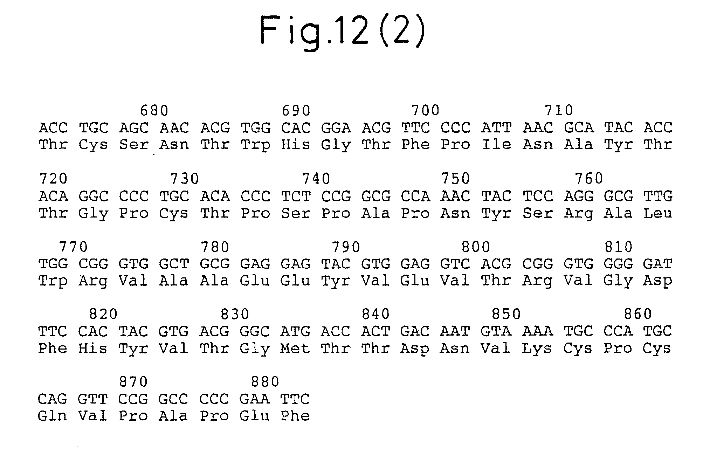

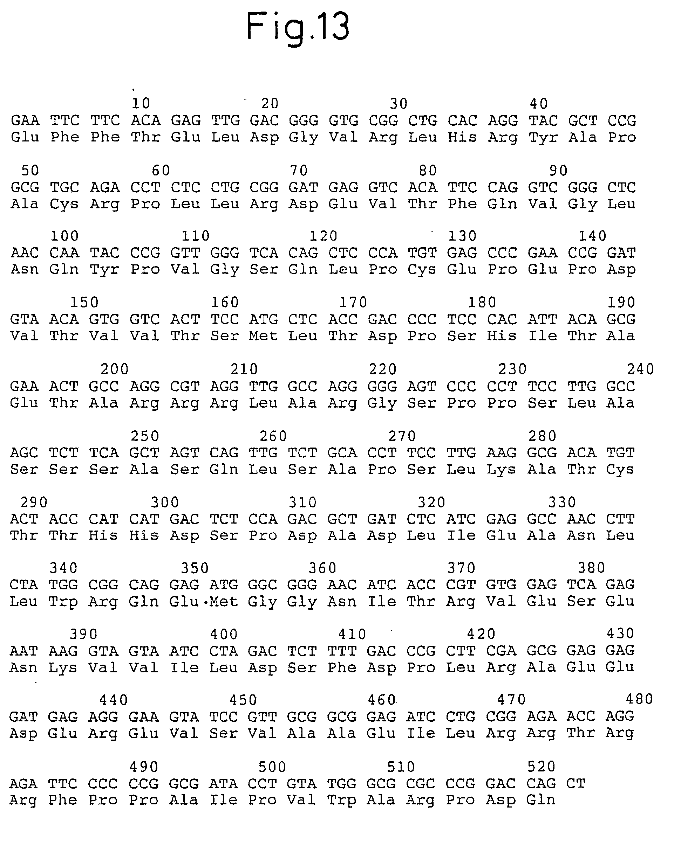

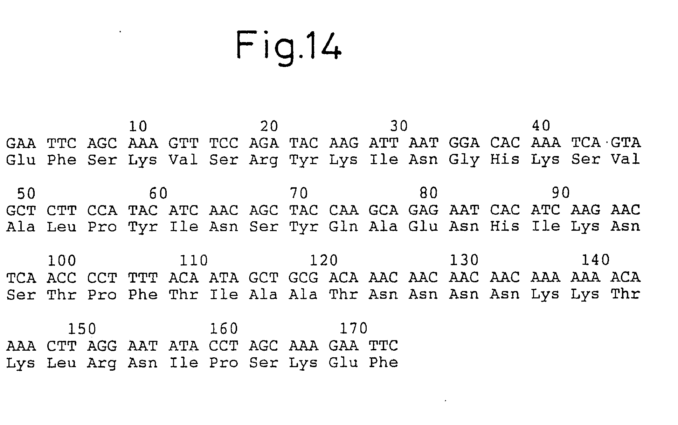

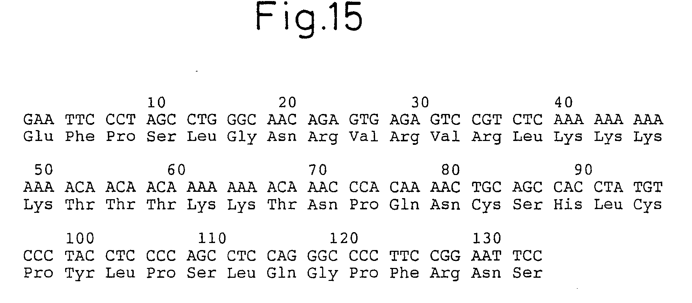

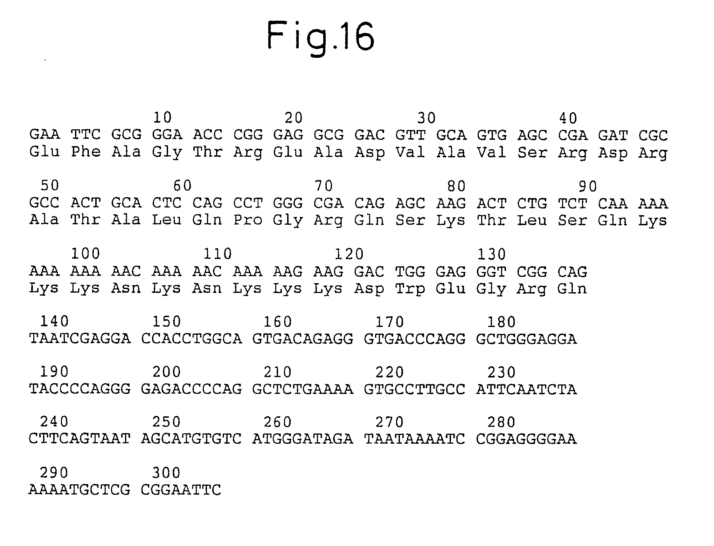

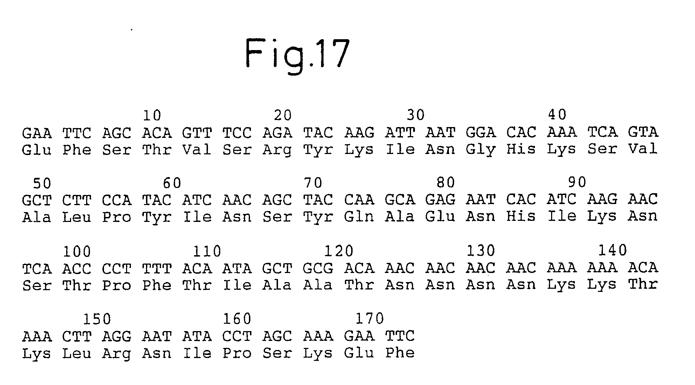

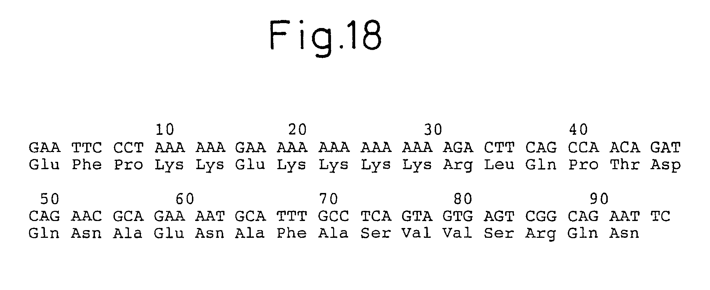

The determined DNA sequences of the cDNAs are shown in Figs. 3 - 18, respectively.

Example 7 Expression of the cDNAs coding for the blood-borne non-A, non-B hepatitis specific antigenic protein in E. coli

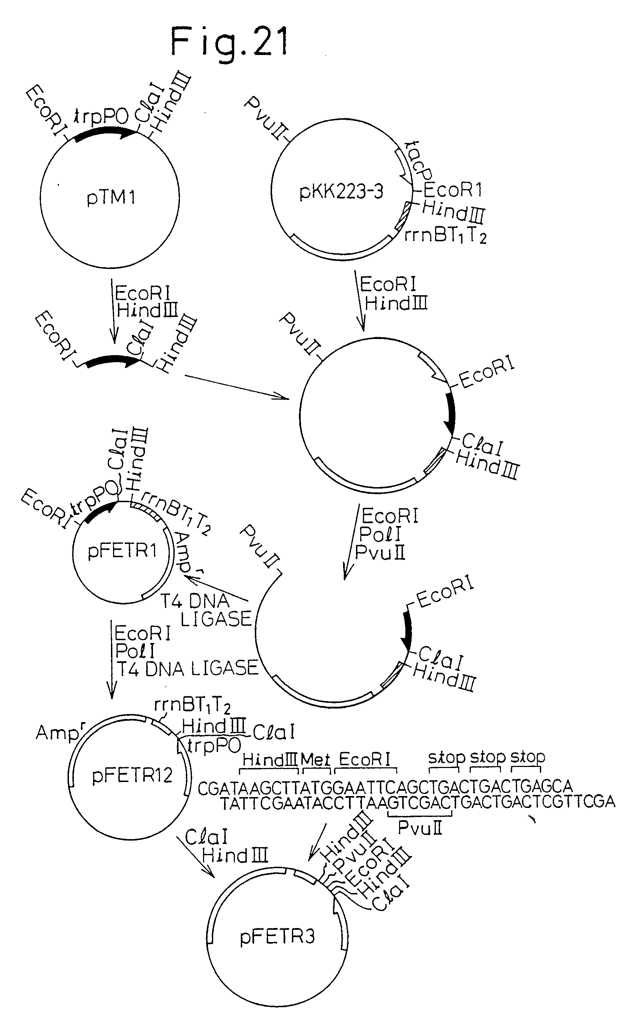

(1) Construction of the expression vector pFETR3 (Fig. 21)

Plasmid pTM1 wherein the 331 bp DNA fragment containing a tryptophan (trp) promoter from the Hpa II site 310 bp upstream of the transcriptional initiation site to the Taq I site 21 bp downstream of the transcriptional initiation site of the E . coli trp operon, prepared from trp transducing phage λcI857 trpED10 [G.F. Miozzari et al., J. Bacteriology, 133 , 1457 (1978)] had been inserted into the Cla I site of pBR322, was digested with EcoR I and Hind III, and ca. 0.4 kb DNA fragment containing the trp promoter was recovered by low-melting agarose gel electrophoresis. pKK223-3 (Pharmacia Co.) was digested with EcoR I and Hind III to give a 4.5 kb DNA fragment, to which the aforementioned ca. 0.4 kb DNA fragment was then ligated using T4 DNA ligase. The plasmid thus obtained was digested with EcoR I and blunted with the E. coli DNA polymerase I (Klenow fragment), and digested with Pvu II. The ca. 3 kb DNA fragment thus obtained was cyclized using the T4 DNA ligase to give a plasmid pFETR1.

The plasmid pFETR1 was digested with EcoR I, made the ends of the DNA fragment were blunted with the E. coli DNA polymerase I (Klenow fragment) and then cyclized using T4 DNA ligase, to give a plasmid pFETR12 containing trp promoter wherein the EcoR I site had been deleted.

To give the translational initiation codon (ATG) and the termination codon (TGA) and the cloning site downstream of the trp promoter on the plasmid pFRTR12, oligonucleotides of 36-mer and 38-mer were synthesized by an automatic DNA synthesizer (380A/APPLIED BIOSYSTEMS CO.) and annealed to give the following DNA linker.

```
                 HindIIIMetEcoRI     stopstopstop
38mer 5' CGATAAGCTTATGGAATTCAGCTGACTGACTGAGCA      3'
36mer 3'   TATTCGAATACCTTAAGTCGACTGACTGACTCGTTCGA 5'
                                Pvu II
```

To the DNA fragment obtained by digesting the aforementioned plasmid pFETR12 with Cla I and Hind III was ligated the aforementioned synthetic DNA linker using the T4 DNA ligase, to give an expression vector pFETR3 containing a trp promoter.

(2) Expression of cDNA in the recombinant phage λHC2207 in E. coli

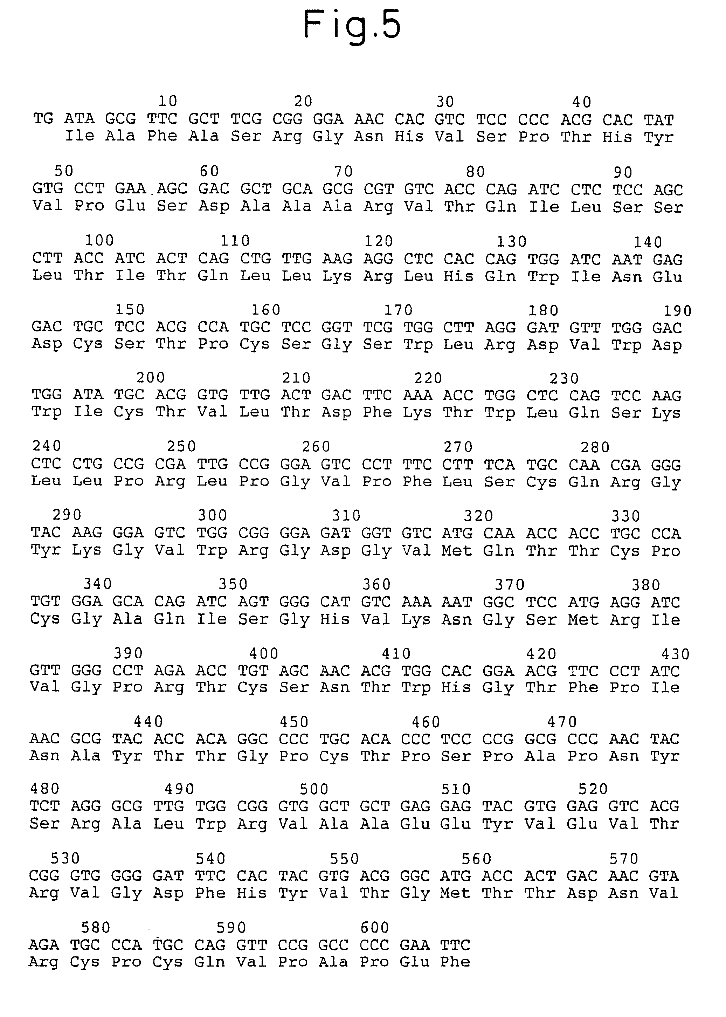

As described in Example 6, the plasmid (referred to as pMC24 hereinafter) obtained by inserting the cDNA of λHC2207 (Fig. 5) into the Hinc II site of pUC19 was digested with Pst I and EcoR I, and the about 0.6 kb DNA fragment containing cDNA was obtained by low-melting agarose gel electrophoresis. Since this DNA fragment has been deleted a part of the 5′-end of the cDNA, oligonucleotides of 79 mer and 73 mer were synthesized on the basis of the amino sequence encoded by the cDNA portion corresponding to the deleted region using the DNA synthesizer (380A/APPLIED BIOSYSTEMS CO.), phosphorylated with T4 polynucleotide kinase, and then annealed to give the following DNA linker:

```
                  10          20          30          40
79mer 5' CGATAAGCTTATGATAGCATTTGCAAGTAGAGGAAATCAC
73mer 3'   TATTCGAATACTATCGTAAACGTTCATCTCCTTTAGTG
         L____JL____JM  I  A  F  A  S  R  G  N  H
         ClaI HindIII

                  50          60          70
         GTGTCCCCCACGCACTATGTGCCTGAAAGCGACGCTGCA 3'
         CACAGGGGGTGCGTCATACACGGACTTTCGCTGCG     5'
         V  S  P  T  H  Y  V  P  E  S  D  A  A
                                          L____J
                                          PstI
```

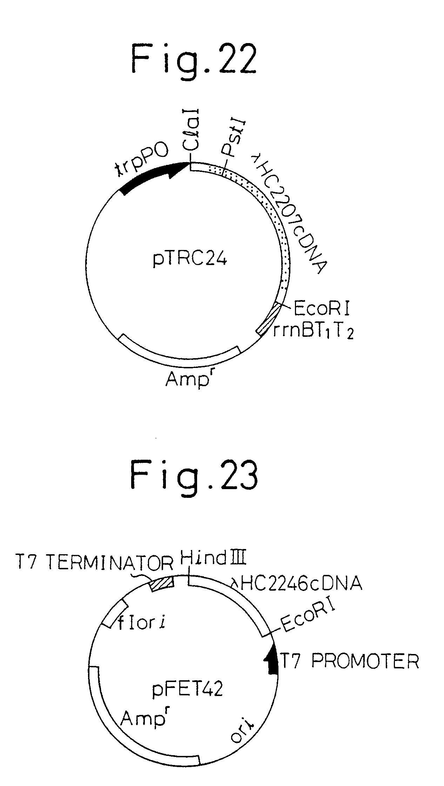

The aforementioned plasmid pFETR3 was digested with Cla I and EcoR I, and the obtained DNA fragment was ligated to the aforementioned synthetic linker and the aforementioned 0.6 kb DNA fragment, to give an expression vector pTRC24 containing a trp promoter and λHC2207 cDNA under the control of said promoter. The structure of this plasmid is shown in Fig. 22.

The E. coli W3110 strain was transformed in the conventional manner with pTRC24. The transformed strain was inoculated in a 2 x TY medium (1.6% bacto-trypton, 1% yeast extract, 0.5% NaCl) containing tryptophan (100 μg/ml) and ampicillin (50 μg/ml), and cultured by shaking at 37°C for 12 - 18 hours. A 40 ml portion of the culture was inoculated in 1 liter of the M9 medium (0.6% $Na_2HPO_4$ , 0.3% $KH_2PO_4$ , 0.1% $NH_4Cl$, 0.05% NaCl, 1 mM $MgCl_2$ , 0.1 mM $CaC_2$) containing 0.8% glucose, 0.5% casamino acid, 10 μg/ml of thiamin hydrochloride, and 50 μg/ml of ampicillin and cultured by shaking at 37°C for ca. 6 hours.

The cells were collected by centrifugation, suspended in a buffer containing 2% sodium dodecylsulfate (SDS) and 500 mM 2-mercaptoethanol, solubilized by boiling, and subjected to SDS-polyacrylamid gel electrophoresis in the conventional manner. The proteins were blotted on a nitrocellulose filter using a semidry blotting apparatus (ATTO Co.), and the reacted with the antibody prepared from the serum of the patient with non-A, non-B hepatitis as described in Example 3. As a result, it was confirmed that the expression product reacted with the antibody.

(3) Expression of the cDNA in the recombinant phage λHC2246 in E. coli

As described in Example 5, the plasmid (referred to as pMC42 hereinafter) obtained by inserting the cDNA of λHC2246 (Fig. 2) into the Sma I site of pUC18 was digested with EcoR I and Pvu II, and subjected to low-melting agarose gel electrophoresis, to give the ca. 0.5 kb DNA fragment containing the cDNA. This DNA fragment was ligated to the EcoR I/Pvu II site of pFETR3 using the T4 DNA ligase, to give pFETR342.

The plasmid pGEMEXTM-1 containing a T7 promoter (PROMEGA Co.) was digested with Xba I and Hind III and subjected to low-melting agarose gel electrophoresis to give the ca. 3.1 kb DNA fragment containing the T7 promoter. To provide the cloning site downstream of the T7 promoter, two kinds of 57 mer oligonucleotides were synthesized by the automatic DNA synthesizer (APPLIED BIOSYSTEMS CO.), and annealed to give the following linker.

```
57mer 5' CTAGAAATAATTTTGTTTAACTTTAAGAAGGAG
57mer 3'     TTTATTAAAACAAATTGAAATTCTTCCTC

                     Met EcoR I Hpa I
                     ┌─┐ ┌───┐ ┌───┐
         ATATACATATGGAATTCGTTAACA      3'
         TATATGTATACCTTAAGCAATTGTTCGA  5'
```

The aforementioned ca. 3.1 kb DNA fragment and the above DNA linker were ligated using T4 DNA ligase, to give a plasmid pFET710 having the translational initiation codon(ATG) and the cloning site downstream of the T7 promoter.

pFETR342 was digested with EcoR I and Hind III and subjected to low-melting agarose gel electrophoresis, to give the ca. 0.5 kb DNA fragment containing the cDNA and the translational termination codon. On the other hand, a DNA fragment was obtained by digesting pFET710 with EcoR I and Hind III. This DNA fragment and the ca. 0.5 kb DNA fragment were ligated using T4 DNA ligase, to give an expression vector pFET42 containing a T7 promoter and λHC2246 cDNA under the control of said promoter. The structure of the vector is shown in Fig. 23.

The E. coli JM109 (DE3) strain (PROMEGA Co.), wherein the expression of the T7 RNA polymerase can be induced with IPTG, was transformed with PFET42. The transformed strain was inoculated in the L medium (1% bacto-trypton, 0.5% yeast extract, 0.5% NaCl) containing ampicillin (50 µg/ml) and cultured by shaking at 30° C for 12 - 18 hours. A 1 ml portion of the culture was inoculated in 100 ml of the L medium containing ampicillin (50 µg/ml), and subjected to shake culture at 30° C. When $A_{660}$ reached ca. 0.3, 0.5 mM IPTG was added, and the culture was further continued for 3 - 5 hours. The bacterial cell obtained was treated in the same manner as described above (2), and it was confirmed that the expression product reacted with the antibody prepared from the serum of the patient with non-A, non-B hepatitis.

(4) The expression of cDNA in λHC2207 and cDNA in λHC2246 in E. coli was confirmed in the above-mentioned (2) and (3), and it is apparent that any cDNAs in other λHC recombinant vectors can be expressed to give an expression product thereof.

As illustrated in (2) and (3), the expression products of the cDNAs in λHC2207 and λHC2246 clones obtained in Example 3 reacted with the serum of the patient with non-A, non-B hepatitis, as when using the recombinant phage in Example 3. Therefore, it has been confirmed that the expression product of the DNA which encodes a non-A, non-B hepatitis specific antigenic protein according to the present invention can be used for the detection of an antibody to a non-A, non-B hepatitis specific antigen.

## Industrial Applicability

The DNA coding for the blood-borne non-A, non-B hepatitis specific antigenic protein according to the present invention is useful as a gene for producing said antigen. Also, the antigenic protein as the expression product of the gene reacts with a antibody in serum against the hepatitis specific antigen, therefore, said antigenic protein is useful as a reagent for the diagnosis of the presence of the antibody in the serum of a subject.

## Claims

1. A DNA coding for a blood-borne non-A, non-B hepatitis specific antigenic protein which appears in a human liver cell and/or blood on the pathopoiesis of the blood-borne non-A, non-B hepatitis.

2. A DNA according to claim 1, wherein said DNA is a cDNA corresponding to RNA extracted from the liver cell of a human patient suffering from the blood-borne non-A, non-B hepatitis.

3. A DNA according to claim 1, wherein said DNA is the cDNA corresponding to RNA extracted from the blood of a human patient suffering from the blood-borne non-A, non-B hepatitis.

4. A DNA according to claims 2 or 3, wherein said DNA is a cDNA inserted to the following phage clone, or contains said cDNA:

| Phage Clone No. | National Deposit No. (FERM P.) | International Deposit No. (FERM BP.) |
|---|---|---|
| λHC 432 | 10897 | |
| λHC 436 | 10898 | |
| λHC 512 | 10841 | 2951 |
| λHC 522 | 10842 | |
| λHC 524 | 10843 | |
| λHC 526 | 10844 | |
| λHC 2206 | 10845 | 2952 |
| λHC 2207 | 10846 | 2953 |
| λHC 2211 | 10876 | 2956 |
| λHC 2216 | 10877 | 2957 |
| λHC 2217 | 10852 | |
| λHC 2220 | 10853 | 2954 |
| λHC 2225 | 10854 | |
| λHC 2230 | 10916 | 2966 |
| λHC 2232 | 10930 | 2968 |
| λHC 2239 | 10931 | |
| λHC 2240 | 10855 | |
| λHC 2241 | 10856 | |
| λHC 2242 | 10857 | |
| λHC 2243 | 10878 | |
| λHC 2244 | 10879 | 2958 |
| λHC 2246 | 10858 | 2955 |

| Phage Clone No. | National Deposit No. (FERM P.) | International Deposit No. (FERM BP.) |
|---|---|---|
| λHC 2248 | 10880 | 2959 |
| λHC 2249 | 10917 | |
| λHC 2250 | 10904 | |
| λHC 2252 | 10881 | |
| λHC 2255 | 10889 | |
| λHC 2256 | 10890 | 2960 |
| λHC 2258 | 10891 | 2961 |
| λHC 2259 | 10892 | |
| λHC 2263 | 10893 | |
| λHC 2264 | 10932 | |
| λHC 2265 | 10933 | |
| λHC 2268 | 10894 | |
| λHC 2270 | 10895 | 2962 |
| λHC 2271 | 10896 | |
| λHC 2404C | 10899 | |
| λHC 2405B | 10900 | |
| λHC 2410A | 10905 | |
| λHC 2410C | 10918 | 2967 |
| λHC 2410D | 10934 | |
| λHC 2413 | 10919 | |
| λHC 2414A | 10906 | |
| λHC 2424A | 10911 | |

| Phage Clone No. | National Deposit No. (FERM P.) | International Deposit No. (FERM BP.) |
|---|---|---|
| λHC 2501 | 10920 | |
| λHC 2502 | 10847 | |
| λHC 2505 | 10921 | |
| λHC 2507 | 10935 | |
| λHC 2508 | 10859 | |
| λHC 2509 | 10936 | |
| λHC 2512 | 10882 | |
| λHC 2514 | 10860 | |
| λHC 2516 | 10861 | |
| λHC 2533 | 10907 | 2963 |
| λHC 2534 | 10937 | |
| λHC 2535 | 10883 | |
| λHC 2602 | 10908 | |
| λHC 2603B | 10922 | |
| λHC 2607 | 10909 | 2964 |
| λHC 2608 | 10923 | |
| λHC 2610 | 10910 | 2965 |

5. A DNA coding for an amino acid sequence encoded by the DNA according to claims 2 or 3 with a different codon.
6. A DNA having a nucleotide sequence according to any one of Figs. 1 - 18.
7. A DNA coding for an amino acid sequence according to any one of Figs. 3 - 20.
8. A blood-borne non-A, non-B hepatitis specific antigenic protein which appears in a human liver cell and/or blood on the pathopoiesis of the blood-borne non-A, non-B hepatitis, characterized by being produced with a DNA coding for said protein by a genetic recombination technique.
9. A protein according to claim 8, wherein said protein has an amino acid sequence described in any one of Figs. 3 - 20.
10. A process for producing a blood-borne non-A, non-B hepatitis specific antigenic protein which appears in a human liver cell and/or blood on the pathopoiesis of the blood-borne non-A, non-B hepatitis, characterized in that a host transformed with an expression vector containing a DNA coding for said protein is cultured.
11. A process according to claim 10, wherein said expression vector is a plasmid and said host is Escherichia coli.
12. A recombinant DNA molecule for use in the cloning of DNA in bacteria, yeast or animal cells, which is
(a) a cDNA inserted into the phage clones listed below;

(b) a DNA which can be hybridized with said cDNA and encodes a blood-borne non-A, non-B hepatitis specific antigenic protein; or
(c) a DNA coding for an amino acid sequence encoded by the DNA described in said (a) or (b) with a different codon:

| Phage Clone No. | National Deposit No. (FERM P.) | International Deposit No. (FERM BP.) |
|---|---|---|
| λHC 432 | 10897 | |
| λHC 436 | 10898 | |
| λHC 512 | 10841 | 2951 |
| λHC 522 | 10842 | |
| λHC 524 | 10843 | |
| λHC 526 | 10844 | |
| λHC 2206 | 10845 | 2952 |
| λHC 2207 | 10846 | 2953 |
| λHC 2211 | 10876 | 2956 |
| λHC 2216 | 10877 | 2957 |
| λHC 2217 | 10852 | |
| λHC 2220 | 10853 | 2954 |
| λHC 2225 | 10854 | |
| λHC 2230 | 10916 | 2966 |
| λHC 2232 | 10930 | 2968 |
| λHC 2239 | 10931 | |
| λHC 2240 | 10855 | |
| λHC 2241 | 10856 | |
| λHC 2242 | 10857 | |
| λHC 2243 | 10878 | |
| λHC 2244 | 10879 | 2958 |
| λHC 2246 | 10858 | 2955 |

| Phage Clone No. | National Deposit No. (FERM P.) | International Deposit No. (FERM BP.) |
|---|---|---|
| λHC 2248 | 10880 | 2959 |
| λHC 2249 | 10917 | |
| λHC 2250 | 10904 | |
| λHC 2252 | 10881 | |
| λHC 2255 | 10889 | |
| λHC 2256 | 10890 | 2960 |
| λHC 2258 | 10891 | 2961 |
| λHC 2259 | 10892 | |
| λHC 2263 | 10893 | |
| λHC 2264 | 10932 | |
| λHC 2265 | 10933 | |
| λHC 2268 | 10894 | |
| λHC 2270 | 10895 | 2962 |
| λHC 2271 | 10896 | |
| λHC 2404C | 10899 | |
| λHC 2405B | 10900 | |
| λHC 2410A | 10905 | |
| λHC 2410C | 10918 | 2967 |
| λHC 2410D | 10934 | |
| λHC 2413 | 10919 | |
| λHC 2414A | 10906 | |
| λHC 2424A | 10911 | |

| Phage Clone No. | National Deposit No. (FERM P.) | International Deposit No. (FERM BP.) |
|---|---|---|
| λHC 2501 | 10920 | |
| λHC 2502 | 10847 | |
| λHC 2505 | 10921 | |
| λHC 2507 | 10935 | |
| λHC 2508 | 10859 | |
| λHC 2509 | 10936 | |
| λHC 2512 | 10882 | |
| λHC 2514 | 10860 | |
| λHC 2516 | 10861 | |
| λHC 2533 | 10907 | 2963 |
| λHC 2534 | 10937 | |
| λHC 2535 | 10883 | |
| λHC 2602 | 10908 | |
| λHC 2603B | 10922 | |
| λHC 2607 | 10909 | 2964 |
| λHC 2608 | 10923 | |
| λHC 2610 | 10910 | 2965 |

SEQUENCE LISTING

SEQ ID NO.: 1

SEQUENCE TYPE: Nucleotide

SEQUENCE LENGTH: 668 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAATTCTTCA CGGAATTGGA TGGGGTGCGG CTACACAGGT ACGCTCCGGC  50
GTGCAAGCCT CTCCTACGGG ATGAGGTCAC ATTCCAGGTC GGGCTCAACC 100
AATTCCCGGT TGGGTCACAG CTCCCATGTG AACCCGAACC GGATGTAATG 150
GTGGTCACCT CTATGCTCAC CGACCCCTCC CATATTACAG CAGAGACGGC 200
TAAGCGTAGG CTGGCCAGAG GGTCTCCCCC TTCTTTGGCC AGCTCTTCAG 250
CTAGTCAGTT GTCTGCGCCC TCCTTGAAGG CGACATGCAC CACCCGTCAT 300
GACTCCCCGG ACGCTGACCT CATAGAGGCC AACCTCCTGT GGCGGCAGGA 350
GATGGGCGGG AACATCACCC GTGTGGAGTC AGAGAATAAG GTAGTGATTT 400
TGGACTCTTT TGAACCGCTT CGGGTGGAGG AGGATGAGAG GGAAGTATCC 450
GTAGCGGCGG ATTTCAGTGA CTTGAATGCA GAATGAATCC CGTGGCTCAC 500
TTCCTAGACT ATTTGCCAAA GAAGATGTTG CGCTGGCCAT GATCAAGATG 550
ACACAAACGG TGGCCTTTTG CAGGGAGAAC CGCCGTGGAG GCCTGTGTCT 600
GTGGCACTGG TAGCTTCTCT CTGCAGGCAA AGACCCCATG GCTTAGTTCT 650
TCATCAGAGT GAGAATTC                                    668
```

SEQ ID NO.: 2

SEQUENCE TYPE: Nucleotide

SEQUENCE LENGTH: 479 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAATTCTTCA CGGAGTTGGA TGGGGTACGG CTGCACAGGT ACGCTCCGGC  50
GTGCAAGCCA CTCCTACGGG ATGAGGTCAC ATTCCAGGTC GGGCTCAACC 100
AATTTCCAGT TGGATCACAG CTCCCATGTG AGCCCGAGCC GGATGTAGCG 150
GTGCTCACTT CCATGCTCAC CGACCCCTCC CACATTACAG CAGAGACGGC 200
TAAGCGTAGG CTGGCCAGGG GGTCCCCCCC CTCCTTGGCC AGCTCTTCAG 250
CTAGTCAGTT GTCTGCGCCC TCCTTGAAGG CGACATGCAC TACCCACCAT 300
GACTCCCCGG ACGCTGACCT CATCGAGGCC AACCTCCTGT GGCGGCAGGA 350
GATGGGAGGA AACATCACCC GCGTGGAGTC AGAGAATAAG GTAGTAATTC 400
TAGACTCTTT TGACCCGCTC CGAGCGGAGG AGGATGAGAG GGAAGTGTCC 450
GTTGCGGCGG AGATCCTGCG GAAGACCAG                        479
```

SEQ ID NO.: 3

SEQUENCE TYPE: Nucleotide with corresponding peptide

SEQUENCE LENGTH: 498 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
TCA CTC AAT CCT CGA CGG TGC TGC CGG TGC GGC AAT CCG      39
Ser Leu Asn Pro Arg Arg Cys Cys Arg Cys Gly Asn Pro
                  5                  10

GAA CGA TAC CGA CGC CGG ATC GCC CTG CTG CCC CCA CGC      78
Glu Arg Tyr Arg Arg Arg Ile Ala Leu Leu Pro Pro Arg
     15                  20                  25

ATT TAC CGC CCG GAC TGT CAG CCT GTA GTT CCC CAG CGC     117
Ile Tyr Arg Pro Asp Cys Gln Pro Val Val Pro Gln Arg
             30                  35

CAG TTG CGT GAA GCG GTA TGT GGT TTC CGT CGT CCG GGC     156
Gln Leu Arg Glu Ala Val Cys Gly Phe Arg Arg Pro Gly
 40                  45                  50

CGT GCT GAC CAG CCG CTC ACT GCC GTC GTC CGT GTT ACG     195
Arg Ala Asp Gln Pro Leu Thr Ala Val Val Arg Val Thr
         55                  60                  65

GTC AGA CGG AGC AGG AAA CTC ACG CCT TCA CAC TTC GGT     234
Val Arg Arg Ser Arg Lys Leu Thr Pro Ser His Phe Gly
                 70                  75

GTG TCC CAT CGC GCC AGC ACC TGATATTCCC CGCTGTCTGC      275
Val Ser His Arg Ala Ser Thr
     80                  85

AGTGACTTCT GCGGTCAGGT GCTGCACCGC TCGTGACACC            315

ATTCACCGTG CCACTCTGTT CGCCGTCAAA GTGCGCCCCG            355

TTATCCACGA TGGCCTCTTT TTCCGGCACA TGCTGCACGG            395
```

CGGTGATGGC ATACGTGCCG TCGTCGTTCT CACGGATACT 435

CACGCAGCGG AACAGTCCTG GCGCAGCGTC GGCAGCTTCA 475

GCTCCCATAC GCTGTATTCA GCT 498

SEQ ID NO.: 4

SEQUENCE TYPE: Nucleotide with corresponding peptide

SEQUENCE LENGTH: 685 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAC GGG GTG CGG CTG CAC AGG      39
Glu Phe Phe Thr Glu Leu Asp Gly Val Arg Leu His Arg
                 5                  10

TAC GCT CCG GCG TGC AGA CCT CTC CTG CGG GAT GAG GTC      78
Tyr Ala Pro Ala Cys Arg Pro Leu Leu Arg Asp Glu Val
    15                  20                  25

ACA TTC CAG GTC GGG CTC AAC CAA TAC CCG GTT GGG TCA     117
Thr Phe Gln Val Gly Leu Asn Gln Tyr Pro Val Gly Ser
            30                  35

CCG CTC CCA TGT GAG CCC GAA CCG GAT GTA ACA GTG GTC     156
Pro Leu Pro Cys Glu Pro Glu Pro Asp Val Thr Val Val
 40                 45                  50

ACT TCC ATG CTC ACC GAC CCC TCC CAC ATT ACA GCG GAA     195
Thr Ser Met Leu Thr Asp Pro Ser His Ile Thr Ala Glu
        55                  60                  65

ACT GCC AGG CGT AGG TTG GCC AGG GGG AGT CCC CCT TCC     234
Thr Ala Arg Arg Arg Leu Ala Arg Gly Ser Pro Pro Ser
                70                  75

TTG GCC AGC TCT TCA GCT AGT CAG TTG TCT GCA CCT TCC     273
Leu Ala Ser Ser Ser Ala Ser Gln Leu Ser Ala Pro Ser
    80                  85                  90

TTG AAG GCG ACA TGT ACT ACC CAT CAT GAC TCT CCA GAC     312
Leu Lys Ala Thr Cys Thr Thr His His Asp Ser Pro Asp
            95                 100
```

```
GCT GAT CTC ATC GAG GCC AAC CTT CTA TGG CGG CAG GAG      351
Ala Asp Leu Ile Glu Ala Asn Leu Leu Trp Arg Gln Glu
105                 110                 115

ATG GGC GGG AAC ATC ACC CGT GTG GAG TCA GAG AAT AAG      390
Met Gly Gly Asn Ile Thr Arg Val Glu Ser Glu Asn Lys
        120                 125                 130

GTA GTA ATC CTA GAC TCT TTT GAC CCG CTT CGA GCG GAG      429
Val Val Ile Leu Asp Ser Phe Asp Pro Leu Arg Ala Glu
                135                 140

GAG GAT GAG AGG GAA GTA TCC GTT GCG GCG GAG ATC CTG      468
Glu Asp Glu Arg Glu Val Ser Val Ala Ala Glu Ile Leu
    145                 150                 155

CGG AGA ACC AGG AGA TTC CCC CCG GCG ATA CCT GTA TGG      507
Arg Arg Thr Arg Arg Phe Pro Pro Ala Ile Pro Val Trp
            160                 165

GCG CGC CCG GAC TAC AAC CCG CCA CTG ATA GAA TCT TGG      546
Ala Arg Pro Asp Tyr Asn Pro Pro Leu Ile Glu Ser Trp
170                 175                 180

AAG GAC CCA GAC TAC GTC CCA CCG GTG GTA CAC GGG TGT      585
Lys Asp Pro Asp Tyr Val Pro Pro Val Val His Gly Cys
        185                 190                 195

CCA TTG CCA CCT GCC AAG ACC CCT CAA GTG GAT ATT CAG      624
Pro Leu Pro Pro Ala Lys Thr Pro Gln Val Asp Ile Gln
                200                 205

ACC TCT TTG AGG CTT TCG TTG GAA ACG GGA TTT CTT CAT      663
Thr Ser Leu Arg Leu Ser Leu Glu Thr Gly Phe Leu His
    210                 215                 220

ACT ATG CTA GAC AGA AGA ATT C                            685
Thr Met Leu Asp Arg Arg Ile
            225
```

SEQ ID NO.: 5

SEQUENCE TYPE: Nucleotide with corresponding peptide

SEQUENCE LENGTH: 608 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
TG ATA GCG TTC GCT TCG CGG GGA AAC CAC GTC TCC CCC       38
   Ile Ala Phe Ala Ser Arg Gly Asn His Val Ser Pro
                    5                  10

ACG CAC TAT GTG CCT GAA AGC GAC GCT GCA GCG CGT GTC      77
Thr His Tyr Val Pro Glu Ser Asp Ala Ala Ala Arg Val
         15                  20                  25

ACC CAG ATC CTC TCC AGC CTT ACC ATC ACT CAG CTG TTG     116
Thr Gln Ile Leu Ser Ser Leu Thr Ile Thr Gln Leu Leu
                 30                  35

AAG AGG CTC CAC CAG TGG ATC AAT GAG GAC TGC TCC ACG     155
Lys Arg Leu His Gln Trp Ile Asn Glu Asp Cys Ser Thr
     40                  45                  50

CCA TGC TCC GGT TCG TGG CTT AGG GAT GTT TGG GAC TGG     194
Pro Cys Ser Gly Ser Trp Leu Arg Asp Val Trp Asp Trp
             55                  60

ATA TGC ACG GTG TTG ACT GAC TTC AAA ACC TGG CTC CAG     233
Ile Cys Thr Val Leu Thr Asp Phe Lys Thr Trp Leu Gln
 65                  70                  75

TCC AAG CTC CTG CCG CGA TTG CCG GGA GTC CCT TTC CTT     272
Ser Lys Leu Leu Pro Arg Leu Pro Gly Val Pro Phe Leu
         80                  85                  90

TCA TGC CAA CGA GGG TAC AAG GGA GTC TGG CGG GGA GAT     311
Ser Cys Gln Arg Gly Tyr Lys Gly Val Trp Arg Gly Asp
                 95                 100
```

```
GGT GTC ATG CAA ACC ACC TGC CCA TGT GGA GCA CAG ATC       350
Gly Val Met Gln Thr Thr Cys Pro Cys Gly Ala Gln Ile
    105                 110                 115

AGT GGG CAT GTC AAA AAT GGC TCC ATG AGG ATC GTT GGG       389
Ser Gly His Val Lys Asn Gly Ser Met Arg Ile Val Gly
            120                 125

CCT AGA ACC TGT AGC AAC ACG TGG CAC GGA ACG TTC CCT       428
Pro Arg Thr Cys Ser Asn Thr Trp His Gly Thr Phe Pro
130                 135                 140

ATC AAC GCG TAC ACC ACA GGC CCC TGC ACA CCC TCC CCG       467
Ile Asn Ala Tyr Thr Thr Gly Pro Cys Thr Pro Ser Pro
        145                 150                 155

GCG CCC AAC TAC TCT AGG GCG TTG TGG CGG GTG GCT GCT       506
Ala Pro Asn Tyr Ser Arg Ala Leu Trp Arg Val Ala Ala
                160                 165

GAG GAG TAC GTG GAG GTC ACG CGG GTG GGG GAT TTC CAC       545
Glu Glu Tyr Val Glu Val Thr Arg Val Gly Asp Phe His
    170                 175                 180

TAC GTG ACG GGC ATG ACC ACT GAC AAC GTA AGA TGC CCA       584
Tyr Val Thr Gly Met Thr Thr Asp Asn Val Arg Cys Pro
            185                 190

TGC CAG GTT CCG GCC CCC GAA TTC                           608
Cys Gln Val Pro Ala Pro Glu Phe
195                 200
```

SEQ ID NO.: 6

SEQUENCE TYPE: Nucleotide with corresponding peptide

SEQUENCE LENGTH: 473 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAT GGG GTG CGG TTG CAC AGG     39
Glu Phe Phe Thr Glu Leu Asp Gly Val Arg Leu His Arg
                 5                  10

TAC GCT CCG GCT GCA AAG CCT CTC CTA CGG GAT GAG GTC     78
Tyr Ala Pro Ala Ala Lys Pro Leu Leu Arg Asp Glu Val
     15                  20                  25

ACA TTT CAG GTC GGG CTC AAC CAA TTC CCG GTT GGG TCA    117
Thr Phe Gln Val Gly Leu Asn Gln Phe Pro Val Gly Ser
             30                  35

CAG CTC CCA TGT GAG CCC GAA CCG GAT GTA ACA GTG ATC    156
Gln Leu Pro Cys Glu Pro Glu Pro Asp Val Thr Val Ile
 40                  45                  50

ACC TCC ATG CTC ACC GAC CCC TCC CAC ATT ACA GCA GAG    195
Thr Ser Met Leu Thr Asp Pro Ser His Ile Thr Ala Glu
         55                  60                  65

GCG GCT GGG CGT AGG CTG GCC AGA GGG TCT CCC CCT TCC    234
Ala Ala Gly Arg Arg Leu Ala Arg Gly Ser Pro Pro Ser
                 70                  75

TTG GCC AGC TCT TCG GCT AGT CAG TTG TCT GCG CCC TTC    273
Leu Ala Ser Ser Ser Ala Ser Gln Leu Ser Ala Pro Phe
     80                  85                  90

CCT GTT GAA GGC CGA CAT GCA CTA CCC GTC ATG ACT CCC    312
Pro Val Glu Gly Arg His Ala Leu Pro Val Met Thr Pro
             95                 100
```

```
CAG ACG CTG ACC TCA TCG AGG CCA ATC TCC TGT GGC GGC     351
Gln Thr Leu Thr Ser Ser Arg Pro Ile Ser Cys Gly Gly
105                 110                 115

AGG AGA TGG GAG GGA ACA TCA CCC GCG TGG AGT CAG AGA     390
Arg Arg Trp Glu Gly Thr Ser Pro Ala Trp Ser Gln Arg
        120                 125                 130

ACA AGG TAC TAATCCTAGA CTCTTTTGAC CCGCTCCGAG            429
Thr Arg Tyr

CGGAGGAGGA TGAGAGGGAG ATATCTGTTG CGGCCCAGCT GAGC        473
```

SEQ ID NO.: 7

SEQUENCE TYPE: Nucleotide with corresponding peptide

SEQUENCE LENGTH: 526 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG CTG GAT GGG GTG CGG TTG CAC AGG       39
Glu Phe Phe Thr Glu Leu Asp Gly Val Arg Leu His Arg
                 5                   10

TAC GCT CCG GCG TGC AAG CCT CTC CTA CGG GAT GAG GTC       78
Tyr Ala Pro Ala Cys Lys Pro Leu Leu Arg Asp Glu Val
     15                  20                  25

ACA TTT CAG GTC GGG CTC AAC CAA TTC CCG GTT GGG TCA      117
Thr Phe Gln Val Gly Leu Asn Gln Phe Pro Val Gly Ser
             30                  35

CAG CTC CCG TGT GAG CCC GAA CCG GAT GTA ACG GTG ATC      156
Gln Leu Pro Cys Glu Pro Glu Pro Asp Val Thr Val Ile
 40                  45                  50

ACT TCC ATG CTC ACC GAC CCC TCC CAC ATT ACA GCA GAG      195
Thr Ser Met Leu Thr Asp Pro Ser His Ile Thr Ala Glu
         55                  60                  65

ACG GCT GGG CGT AGG CTG GCC AGA GGG TCT CCC CCT TCC      234
Thr Ala Gly Arg Arg Leu Ala Arg Gly Ser Pro Pro Ser
                 70                  75

TTG GCC AGC TCT TCG GCT AGT CAG TTG TCT GCG CCC TCC      273
Leu Ala Ser Ser Ser Ala Ser Gln Leu Ser Ala Pro Ser
     80                  85                  90

TTG AAG GCA ACA TGC ACT ACC CGT CAT GAC TCC CCA GAC      312
Leu Lys Ala Thr Cys Thr Thr Arg His Asp Ser Pro Asp
             95                 100
```

```
GCT GAC CTC ATC GAG GCC AAT CTC CTG TGG CGG CAG GAG      351
Ala Asp Leu Ile Glu Ala Asn Leu Leu Trp Arg Gln Glu
105                 110                 115

ATG GGA GGG AAC ATC ACC CGC GTG GAG TCA GAG AAC AAG      390
Met Gly Gly Asn Ile Thr Arg Val Glu Ser Glu Asn Lys
        120                 125                 130

GTA GTA ATC CTA GAC TCT TTT GAC CCG CTC CGA GCG GAG      429
Val Val Ile Leu Asp Ser Phe Asp Pro Leu Arg Ala Glu
                135                 140

GAG GAT GAG AGG GAG ATA TCT GTT GCG GCG GAG ATC CTA      468
Glu Asp Glu Arg Glu Ile Ser Val Ala Ala Glu Ile Leu
    145                 150                 155

CGG AAA TCT AGG AAA TTC CCC CCA GCA TTA CCC ATA TGG      507
Arg Lys Ser Arg Lys Phe Pro Pro Ala Leu Pro Ile Trp
            160                 165

GCG CGC CCG GAC TAC AACC                                 526
Ala Arg Pro Asp Tyr Asn
170                 175
```

SEQ ID NO.: 8

SEQUENCE TYPE: Nucleotide with corresponding peptide

SEQUENCE LENGTH: 599 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAT GGG GTG CGG CTG CAC AGG      39
Glu Phe Phe Thr Glu Leu Asp Gly Val Arg Leu His Arg
                  5                  10

TAC GCT CCG GCG TGC AAA CCT CTC CTG CGG GAT GAG GTC      78
Tyr Ala Pro Ala Cys Lys Pro Leu Leu Arg Asp Glu Val
     15                  20                  25

ACG TTC CAG GTC GGG CTC AAC CAA TAC CCG GTT GGA TCA     117
Thr Phe Gln Val Gly Leu Asn Gln Tyr Pro Val Gly Ser
             30                  35

CAG CTC CCA TGC GAG CCC GAA CCG GAT GTG GCG GTG CTC     156
Gln Leu Pro Cys Glu Pro Glu Pro Asp Val Ala Val Leu
 40                  45                  50

ACT TCC ATG CTC ACC GAC CCC ACC CAC ATT ACA GCA GAA     195
Thr Ser Met Leu Thr Asp Pro Thr His Ile Thr Ala Glu
         55                  60                  65

GCG GCT AGG CGC AGG CTG GCC AGA GGG TCT CCT CCT TCC     234
Ala Ala Arg Arg Arg Leu Ala Arg Gly Ser Pro Pro Ser
                 70                  75

TTG GCC AGC TCT TCA GCT AGT CAG TTG TCT GCG CCC TCC     273
Leu Ala Ser Ser Ser Ala Ser Gln Leu Ser Ala Pro Ser
     80                  85                  90

TTG AAG GCG ACA TGC ACT ACC CAT CAT GAC TCC CCA GAC     312
Leu Lys Ala Thr Cys Thr Thr His His Asp Ser Pro Asp
             95                 100
```

```
GCT GAC CTC ATC GAG GCC AAC CTC CTG TGG CGG CAG GAG     351
Ala Asp Leu Ile Glu Ala Asn Leu Leu Trp Arg Gln Glu
105                 110                 115

ATG GGC GGA AAC ATC ACC CGC GTG GAG TCA GAG AAT AAG     390
Met Gly Gly Asn Ile Thr Arg Val Glu Ser Glu Asn Lys
        120                 125                 130

GTA GTA ATT CTA GAC TCT TTT GAA CCG CTT CGA GCG GAA     429
Val Val Ile Leu Asp Ser Phe Glu Pro Leu Arg Ala Glu
                135                 140

GAG GAT GAG AGG GAA GTA TCC GTT GCG GCA GAG ATC CTG     468
Glu Asp Glu Arg Glu Val Ser Val Ala Ala Glu Ile Leu
    145                 150                 155

CGG AAA ACC AGG AGA TTC CCC GCA GCG ATG CCC ATA TGG     507
Arg Lys Thr Arg Arg Phe Pro Ala Ala Met Pro Ile Trp
            160                 165

GCA CGT CCG GAC TAC AAC CCA CCA TTA CTA CAG TCC TGG     546
Ala Arg Pro Asp Tyr Asn Pro Pro Leu Leu Gln Ser Trp
170                 175                 180

AAG GAC CCG GAC TAC GTC CCT CCG GTG GTG CAC GGG TGC     585
Lys Asp Pro Asp Tyr Val Pro Pro Val Val His Gly Cys
        185                 190                 195

CCA TTG CCA CCT GC                                      599
Pro Leu Pro Pro
```

SEQ ID NO.: 9

SEQUENCE TYPE: Nucleotide with corresponding peptide

SEQUENCE LENGTH: 1184 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAT GGG GTG CGG CTG CAC AGG       39
Glu Phe Phe Thr Glu Leu Asp Gly Val Arg Leu His Arg
                 5                  10

TAC GCT CCG GCG TGC AAA CCT CTC CTG CGG GAT GAG GTC       78
Tyr Ala Pro Ala Cys Lys Pro Leu Leu Arg Asp Glu Val
     15                  20                  25

ACG TTC CAG GTC GGG CTC AAC CAA TAC CCG GTT GGA TCA      117
Thr Phe Gln Val Gly Leu Asn Gln Tyr Pro Val Gly Ser
             30                  35

CAG CTC CCA TGC GAG CCC GAA CCG GAT GTG GCG GTG CTC      156
Gln Leu Pro Cys Glu Pro Glu Pro Asp Val Ala Val Leu
 40                  45                  50

ACT TCC ATG CTC ACC GAC CCC ACC CAC ATT ACA GCA GAA      195
Thr Ser Met Leu Thr Asp Pro Thr His Ile Thr Ala Glu
         55                  60                  65

GCG GCT AGG CGC AGG CTG GCC AGA GGG TCT CCT CCT TCC      234
Ala Ala Arg Arg Arg Leu Ala Arg Gly Ser Pro Pro Ser
                 70                  75

TTG GCC AGC TCT TCA GCT AGT CAG TTG TCT GCG CCC TCC      273
Leu Ala Ser Ser Ser Ala Ser Gln Leu Ser Ala Pro Ser
     80                  85                  90

TTG AAG GCG ACA TGC ACT ACC CAT CAT GAC TCC CCA GAC      312
Leu Lys Ala Thr Cys Thr Thr His His Asp Ser Pro Asp
             95                 100
```

```
GCT GAC CTC ATC GAG GCC AAC CTC CTG TGG CGG CAG GAG      351
Ala Asp Leu Ile Glu Ala Asn Leu Leu Trp Arg Gln Glu
105                 110                 115

ATG GGC GGA AAC ATC ACC CGC GTG GAG TCA GAG AAT AAG      390
Met Gly Gly Asn Ile Thr Arg Val Glu Ser Glu Asn Lys
        120                 125                 130

GTA GTA ATT CTA GAC TCT TTT GAA CCG CTT CGA GCG GAA      429
Val Val Ile Leu Asp Ser Phe Glu Pro Leu Arg Ala Glu
                135                 140

GAG GAT GAG AGG GAA GTA TCC GTT GCG GCA GAG ATC CTG      468
Glu Asp Glu Arg Glu Val Ser Val Ala Ala Glu Ile Leu
    145                 150                 155

CGG AAA ACC AGG AGA TTC CCC GCA GCG ATG CCC ATA TGG      507
Arg Lys Thr Arg Arg Phe Pro Ala Ala Met Pro Ile Trp
            160                 165

GCA CGT CCG GAC TAC AAC CCA CCA TTA CTA CAG TCC TGG      546
Ala Arg Pro Asp Tyr Asn Pro Pro Leu Leu Gln Ser Trp
170                 175                 180

AAG GAC CCG GAC TAC GTC CCT CCG GTG GTG CAC GGG TGC      585
Lys Asp Pro Asp Tyr Val Pro Pro Val Val His Gly Cys
        185                 190                 195

CCA TTG CCA CCT GCC AAG GCC CCT CCA GTA CCA CCT CCA      624
Pro Leu Pro Pro Ala Lys Ala Pro Pro Val Pro Pro Pro
                200                 205

AGG AGA AAG AGG ACG GTT GTC CTG ACA GAA TCC ACC GTG      663
Arg Arg Lys Arg Thr Val Val Leu Thr Glu Ser Thr Val
    210                 215                 220

TCT TCC GCC TTG GCG GAG CTT GCT ACA AAG ACC TTC GGC      702
Ser Ser Ala Leu Ala Glu Leu Ala Thr Lys Thr Phe Gly
            225                 230

GGG TCC GGA TCA TCG GCC GCC GAC AGC GGC ACA GCA AGC      741
Gly Ser Gly Ser Ser Ala Ala Asp Ser Gly Thr Ala Ser
235                 240                 245

GGC CCT CCT GGC CAG GCC TCC GAC GAT GGA GAT ACA GGA      780
Gly Pro Pro Gly Gln Ala Ser Asp Asp Gly Asp Thr Gly
        250                 255                 260

TCC GAC GTT GAG TCG TAC TCC TCC ATG CCC CCC CTT GAG      819
Ser Asp Val Glu Ser Tyr Ser Ser Met Pro Pro Leu Glu
                265                 270
```

GGA GAG CCG GGG GAC CCC GAC CTC AGC GAC GGG TCT TGG 858
Gly Glu Pro Gly Asp Pro Asp Leu Ser Asp Gly Ser Trp
275 280 285

TCT ACT GTG AGT GAG GAG GCT GGT GAG GAT GTC GTC TGC 897
Ser Thr Val Ser Glu Glu Ala Gly Glu Asp Val Val Cys
290 295

TGC TCG ATG TCC TAC ACA TGG ACA GGC GCC TTA ATC ACA 936
Cys Ser Met Ser Tyr Thr Trp Thr Gly Ala Leu Ile Thr
300 305 310

CCA TGC ACC GCG GAG GAG AGC AAG CTG CCC ATC AAC CCG 975
Pro Cys Thr Ala Glu Glu Ser Lys Leu Pro Ile Asn Pro
315 320 325

TTG AGC AAC TCT TTG CTG CGG GCA TCT GCT CGG GCG TAT 1014
Leu Ser Asn Ser Leu Leu Arg Ala Ser Ala Arg Ala Tyr
330 335

CAT CAA CTG ATG AGC AAG AAG GAT ATA ATT CCT ACG CCC 1053
His Gln Leu Met Ser Lys Lys Asp Ile Ile Pro Thr Pro
340 345 350

TCT CAG CCG ATG AAC AGT TGG AAT AGG TTG TTA GCG GTA 1092
Ser Gln Pro Met Asn Ser Trp Asn Arg Leu Leu Ala Val
355 360

ACT AAG ATT AGT ATG GTA ATT AGG AAA ATG AGT AGA TAT 1131
Thr Lys Ile Ser Met Val Ile Arg Lys Met Ser Arg Tyr
365 370 375

TTG AAG AAC TGATTAATGT TTGGGTCTGA GTTTATATAT 1170
Leu Lys Asn
380

CACAGTGAGA ATTC 1184

SEQ ID NO.: 10

SEQUENCE TYPE: Nucleotide with corresponding peptide

SEQUENCE LENGTH: 255 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GGG CAT GTC AAA AAT GGC TCC ATG AGG ATC GTT GGG CCT      39
Gly His Val Lys Asn Gly Ser Met Arg Ile Val Gly Pro
                 5                   10

AGA ACC TGT AGC AAC ACG TGG CAC GGA ACG TTC CCT ATC      78
Arg Thr Cys Ser Asn Thr Trp His Gly Thr Phe Pro Ile
     15                  20                  25

AAC GCG TAC ACC ACA GGC CCC TGC ACA CCC TCC CCG GCG     117
Asn Ala Tyr Thr Thr Gly Pro Cys Thr Pro Ser Pro Ala
             30                  35

CCC AAC TAC TCT AGG GCG TTG TGG CGG GTG GCT GCT GAG     156
Pro Asn Tyr Ser Arg Ala Leu Trp Arg Val Ala Ala Glu
 40                  45                  50

GAG TAC GTG GAG GTC ACG CGG GTG GGG GAT TTC CAC TAC     195
Glu Tyr Val Glu Val Thr Arg Val Gly Asp Phe His Tyr
         55                  60                  65

GTG ACG GGC ATG ACC ACT GAC AAC GTA AGA TGC CCA TGC     234
Val Thr Gly Met Thr Thr Asp Asn Val Arg Cys Pro Cys
                 70                  75

CAG GTT CCG GCC CCC GAA TTC                             255
Gln Val Pro Ala Pro Glu Phe
     80                  85
```

SEQ ID NO.: 11

SEQUENCE TYPE: Nucleotide with corresponding peptide

SEQUENCE LENGTH: 553 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAT GGG GTG CGG TTG CAC AGG      39
Glu Phe Phe Thr Glu Leu Asp Gly Val Arg Leu His Arg
                 5                  10

TAC GCT CCG GCG TGC AAA CCT CTC CTA CGG GAT GAG GTC      78
Tyr Ala Pro Ala Cys Lys Pro Leu Leu Arg Asp Glu Val
     15                  20                  25

ACA TTT CAG GTC GGG CTC AAC CAA TTC CCG GTT GGG TCA     117
Thr Phe Gln Val Gly Leu Asn Gln Phe Pro Val Gly Ser
             30                  35

CAG CTC CCA TGT GAG CCC GAA CCG GAT GTA ACA GTG ATC     156
Gln Leu Pro Cys Glu Pro Glu Pro Asp Val Thr Val Ile
 40                  45                  50

ACC TCC ATG CTC ACC GAC CCC TCC CAC ATT ACA GCA GAG     195
Thr Ser Met Leu Thr Asp Pro Ser His Ile Thr Ala Glu
         55                  60                  65

ACG GCT GGG CGT AGG CTG GCC AGA GGG TCT CCC CCT TCC     234
Thr Ala Gly Arg Arg Leu Ala Arg Gly Ser Pro Pro Ser
                 70                  75

TTG GCC AGC TCT TCG GCT AGT CAG TTG TCT GCG CCT TCC     273
Leu Ala Ser Ser Ser Ala Ser Gln Leu Ser Ala Pro Ser
     80                  85                  90

TTG AAG GCG ACA TGC ACT ACC CGT CAT GAC TCC CCA GAC     312
Leu Lys Ala Thr Cys Thr Thr Arg His Asp Ser Pro Asp
             95                 100
```

```
GCT GAC CTC ATC GAG GCC AAT CTC CTG TGG CGG CAG GAG      351
Ala Asp Leu Ile Glu Ala Asn Leu Leu Trp Arg Gln Glu
105                 110                 115

ATG GGA GGG AAC ATC ACC CGC GTG GAG TCA GAG AAC AAG      390
Met Gly Gly Asn Ile Thr Arg Val Glu Ser Glu Asn Lys
        120                 125                 130

GTA GTA ATC CTA GAC TCT TTT GAC CCG CTC CGA GCG GAG      429
Val Val Ile Leu Asp Ser Phe Asp Pro Leu Arg Ala Glu
                135                 140

GAG GAT GAG AGG GAG ATA TCT GTT GCG GCG GAG ATC CTA      468
Glu Asp Glu Arg Glu Ile Ser Val Ala Ala Glu Ile Leu
    145                 150                 155

CGG AAA TCT AGG AAA TTC CCC CCA GCA TTA CCC ATA TGG      507
Arg Lys Ser Arg Lys Phe Pro Pro Ala Leu Pro Ile Trp
            160                 165

GCG CGC CCG GAC TAC AAC CCA CCA CTG CTA GAG TCT TGG      546
Ala Arg Pro Asp Tyr Asn Pro Pro Leu Leu Glu Ser Trp
170                 175                 180

CCA GCT G                                                553
Pro Ala
```

SEQ ID NO.: 12

SEQUENCE TYPE: Nucleotide with corresponding peptide

SEQUENCE LENGTH: 884 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
CG GCT TTC GTG GGC GCC GGC ATA GCC GGC GCG GCT GTT        38
   Ala Phe Val Gly Ala Gly Ile Ala Gly Ala Ala Val
                   5                  10

GGC AGC ATA GGC CTT GGG AAG GTG CTT GTG GAC ATC CTG       77
Gly Ser Ile Gly Leu Gly Lys Val Leu Val Asp Ile Leu
        15                  20                  25

GCG GGT TAT GGA GCA GGG GTG GCA GGC GCA CTC GTG GTC      116
Ala Gly Tyr Gly Ala Gly Val Ala Gly Ala Leu Val Val
                30                  35

TTT AAG GTT ATG AGT GGC GAC ATG CCC TCC ACC GAG GAC      155
Phe Lys Val Met Ser Gly Asp Met Pro Ser Thr Glu Asp
    40                  45                  50

CTG GTC AAC TTA CTC CCT GCC ATC CTT TCC CCT GGC GCC      194
Leu Val Asn Leu Leu Pro Ala Ile Leu Ser Pro Gly Ala
            55                  60

CTG GTC GTC GGG GTC GTG TGC GCA CAG ATA CTG CGT CGA      233
Leu Val Val Gly Val Val Cys Ala Gln Ile Leu Arg Arg
65                  70                  75

CAT GTC GGC CCA GGG GAG GGA GCT GTG CAG TGG ATG AAC      272
His Val Gly Pro Gly Glu Gly Ala Val Gln Trp Met Asn
        80                  85                  90

CGG CTG ATA GCG TTC GCT TCG CGG GGT AAC CAC GTC TCC      311
Arg Leu Ile Ala Phe Ala Ser Arg Gly Asn His Val Ser
                95                  100
```

```
CCC ACG CAT TAT GTG CCT GAA AGC GAC GCT GCG AGT CGT      350
Pro Thr His Tyr Val Pro Glu Ser Asp Ala Ala Ser Arg
    105                 110                 115

GTC ACC CAG ATC CTC TCC AGC CTT ACC ATC ACT CAG CTG      389
Val Thr Gln Ile Leu Ser Ser Leu Thr Ile Thr Gln Leu
            120                     125

TTG AAG AGG CTC CAC CAG TGG ATT AAT GAG GAC TGC TCC      428
Leu Lys Arg Leu His Gln Trp Ile Asn Glu Asp Cys Ser
130                 135                 140

ACG CCA TGC TCC GGC ACG TGG CTC AGG GAT GTT TGG GAC      467
Thr Pro Cys Ser Gly Thr Trp Leu Arg Asp Val Trp Asp
        145                 150                     155

TGG ATA TGC ACG GTG TTG GCT GAC TTC AAG ACC TGG CTC      506
Trp Ile Cys Thr Val Leu Ala Asp Phe Lys Thr Trp Leu
                160                 165

CAG TCC AAG CTC CTG CCG CGG TTA CCG GGG GTC CCT TTC      545
Gln Ser Lys Leu Leu Pro Arg Leu Pro Gly Val Pro Phe
    170                 175                 180

CTC TCA TGT CAG CGT GGG TAC AAG GGA GTT TGG CGG GGA      584
Leu Ser Cys Gln Arg Gly Tyr Lys Gly Val Trp Arg Gly
            185                     190

GAT GGC ATC ATG CAC ACC ACC TGC CCA TGC GGA GCC CAA      623
Asp Gly Ile Met His Thr Thr Cys Pro Cys Gly Ala Gln
195                 200                 205

ATC ACC GGA CAT GTC AAA AAC GGG TCC ATG AGG ATC GCC      662
Ile Thr Gly His Val Lys Asn Gly Ser Met Arg Ile Ala
        210                 215                     220

GGG CCT AAA ACC TGC AGC AAC ACG TGG CAC GGA ACG TTC      701
Gly Pro Lys Thr Cys Ser Asn Thr Trp His Gly Thr Phe
                225                 230

CCC ATT AAC GCA TAC ACC ACA GGC CCC TGC ACA CCC TCT      740
Pro Ile Asn Ala Tyr Thr Thr Gly Pro Cys Thr Pro Ser
    235                 240                 245

CCG GCG CCA AAC TAC TCC AGG GCG TTG TGG CGG GTG GCT      779
Pro Ala Pro Asn Tyr Ser Arg Ala Leu Trp Arg Val Ala
            250                     255

GCG GAG GAG TAC GTG GAG GTC ACG CGG GTG GGG GAT TTC      818
Ala Glu Glu Tyr Val Glu Val Thr Arg Val Gly Asp Phe
260                 265                 270
```

```
CAC TAC GTG ACG GGC ATG ACC ACT GAC AAT GTA AAA TGC     857
His Tyr Val Thr Gly Met Thr Thr Asp Asn Val Lys Cys
        275                 280                 285

CCA TGC CAG GTT CCG GCC CCC GAA TTC                     884
Pro Cys Gln Val Pro Ala Pro Glu Phe
                290
```

SEQ ID NO.: 13

SEQUENCE TYPE: Nucleotide with corresponding peptide

SEQUENCE LENGTH: 524 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACA GAG TTG GAC GGG GTG CGG CTG CAC AGG      39
Glu Phe Phe Thr Glu Leu Asp Gly Val Arg Leu His Arg
                 5                  10

TAC GCT CCG GCG TGC AGA CCT CTC CTG CGG GAT GAG GTC      78
Tyr Ala Pro Ala Cys Arg Pro Leu Leu Arg Asp Glu Val
     15                  20                  25

ACA TTC CAG GTC GGG CTC AAC CAA TAC CCG GTT GGG TCA     117
Thr Phe Gln Val Gly Leu Asn Gln Tyr Pro Val Gly Ser
             30                  35

CAG CTC CCA TGT GAG CCC GAA CCG GAT GTA ACA GTG GTC     156
Gln Leu Pro Cys Glu Pro Glu Pro Asp Val Thr Val Val
 40                  45                  50

ACT TCC ATG CTC ACC GAC CCC TCC CAC ATT ACA GCG GAA     195
Thr Ser Met Leu Thr Asp Pro Ser His Ile Thr Ala Glu
         55                  60                  65

ACT GCC AGG CGT AGG TTG GCC AGG GGG AGT CCC CCT TCC     234
Thr Ala Arg Arg Arg Leu Ala Arg Gly Ser Pro Pro Ser
                 70                  75

TTG GCC AGC TCT TCA GCT AGT CAG TTG TCT GCA CCT TCC     273
Leu Ala Ser Ser Ser Ala Ser Gln Leu Ser Ala Pro Ser
     80                  85                  90

TTG AAG GCG ACA TGT ACT ACC CAT CAT GAC TCT CCA GAC     312
Leu Lys Ala Thr Cys Thr Thr His His Asp Ser Pro Asp
             95                 100
```

```
GCT GAT CTC ATC GAG GCC AAC CTT CTA TGG CGG CAG GAG      351
Ala Asp Leu Ile Glu Ala Asn Leu Leu Trp Arg Gln Glu
105                 110                 115

ATG GGC GGG AAC ATC ACC CGT GTG GAG TCA GAG AAT AAG      390
Met Gly Gly Asn Ile Thr Arg Val Glu Ser Glu Asn Lys
        120                 125                 130

GTA GTA ATC CTA GAC TCT TTT GAC CCG CTT CGA GCG GAG      429
Val Val Ile Leu Asp Ser Phe Asp Pro Leu Arg Ala Glu
                135                 140

GAG GAT GAG AGG GAA GTA TCC GTT GCG GCG GAG ATC CTG      468
Glu Asp Glu Arg Glu Val Ser Val Ala Ala Glu Ile Leu
    145                 150                 155

CGG AGA ACC AGG AGA TTC CCC CCG GCG ATA CCT GTA TGG      507
Arg Arg Thr Arg Arg Phe Pro Pro Ala Ile Pro Val Trp
            160                 165

GCG CGC CCG GAC CAG CT                                   524
Ala Arg Pro Asp Gln
170
```

SEQ ID NO.: 14

SEQUENCE TYPE: Nucleotide with corresponding peptide

SEQUENCE LENGTH: 174 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC AGC AAA GTT TCC AGA TAC AAG ATT AAT GGA CAC      39
Glu Phe Ser Lys Val Ser Arg Tyr Lys Ile Asn Gly His
                  5                  10

AAA TCA GTA GCT CTT CCA TAC ATC AAC AGC TAC CAA GCA      78
Lys Ser Val Ala Leu Pro Tyr Ile Asn Ser Tyr Gln Ala
     15                  20                  25

GAG AAT CAC ATC AAG AAC TCA ACC CCT TTT ACA ATA GCT     117
Glu Asn His Ile Lys Asn Ser Thr Pro Phe Thr Ile Ala
             30                  35

GCG ACA AAC AAC AAC AAC AAA AAA ACA AAA CTT AGG AAT     156
Ala Thr Asn Asn Asn Asn Lys Lys Thr Lys Leu Arg Asn
 40                  45                  50

ATA CCT AGC AAA GAA TTC                                 174
Ile Pro Ser Lys Glu Phe
         55
```

SEQ ID NO.: 15

SEQUENCE TYPE: Nucleotide with corresponding peptide

SEQUENCE LENGTH: 135 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC CCT AGC CTG GGC AAC AGA GTG AGA GTC CGT CTC      39
Glu Phe Pro Ser Leu Gly Asn Arg Val Arg Val Arg Leu
                 5                  10

AAA AAA AAA AAA ACA ACA ACA AAA AAA ACA AAC CCA CAA      78
Lys Lys Lys Lys Thr Thr Thr Lys Lys Thr Asn Pro Gln
     15                  20                  25

AAC TGC AGC CAC CTA TGT CCC TAC CTC CCC AGC CTC CAG     117
Asn Cys Ser His Leu Cys Pro Tyr Leu Pro Ser Leu Gln
             30                  35

GGC CCC TTC CGG AAT TCC                                 135
Gly Pro Phe Arg Asn Ser
 40                  45
```

SEQ ID NO.: 16

SEQUENCE TYPE: Nucleotide with corresponding peptide

SEQUENCE LENGTH: 306 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC GCG GGA ACC CGG GAG GCG GAC GTT GCA GTG AGC      39
Glu Phe Ala Gly Thr Arg Glu Ala Asp Val Ala Val Ser
                 5                  10

CGA GAT CGC GCC ACT GCA CTC CAG CCT GGG CGA CAG AGC      78
Arg Asp Arg Ala Thr Ala Leu Gln Pro Gly Arg Gln Ser
     15                  20                  25

AAG ACT CTG TCT CAA AAA AAA AAA AAC AAA AAC AAA AAG     117
Lys Thr Leu Ser Gln Lys Lys Lys Asn Lys Asn Lys Lys
             30                  35

AAG GAC TGG GAG GGT CGG CAG TAATCGAGGA CCACCTGGCA       158
Lys Asp Trp Glu Gly Arg Gln
 40                  45

GTGACAGAGG GTGACCCAGG GCTGGGAGGA TACCCCAGGG             198

GAGACCCCAG GCTCTGAAAA GTGCCTTGCC ATTCAATCTA             238

CTTCAGTAAT AGCATGTGTC ATGGGATAGA TAATAAAATC             278

CGGAGGGGAA AAAATGCTCG CGGAATTC                          306
```

SEQ ID NO.: 17

SEQUENCE TYPE: Nucleotide with corresponding peptide

SEQUENCE LENGTH: 174 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC AGC ACA GTT TCC AGA TAC AAG ATT AAT GGA CAC      39
Glu Phe Ser Thr Val Ser Arg Tyr Lys Ile Asn Gly His
                 5                  10

AAA TCA GTA GCT CTT CCA TAC ATC AAC AGC TAC CAA GCA      78
Lys Ser Val Ala Leu Pro Tyr Ile Asn Ser Tyr Gln Ala
     15                  20                  25

GAG AAT CAC ATC AAG AAC TCA ACC CCT TTT ACA ATA GCT     117
Glu Asn His Ile Lys Asn Ser Thr Pro Phe Thr Ile Ala
             30                  35

GCG ACA AAC AAC AAC AAC AAA AAA ACA AAA CTT AGG AAT     156
Ala Thr Asn Asn Asn Asn Lys Lys Thr Lys Leu Arg Asn
 40                  45                  50

ATA CCT AGC AAA GAA TTC                                 174
Ile Pro Ser Lys Glu Phe
         55
```

SEQ ID NO.: 18

SEQUENCE TYPE: Nucleotide with corresponding peptide

SEQUENCE LENGTH: 95 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC CCT AAA AAA GAA AAA AAA AAA AAA AGA CTT CAG      39
Glu Phe Pro Lys Lys Glu Lys Lys Lys Lys Arg Leu Gln
                  5                  10

CCA ACA GAT CAG AAC GCA GAA AAT GCA TTT GCC TCA GTA      78
Pro Thr Asp Gln Asn Ala Glu Asn Ala Phe Ala Ser Val
     15                  20                  25

GTG AGT CGG CAG AAT TC                                   95
Val Ser Arg Gln Asn
             30
```

SEQ ID NO.: 19

SEQUENCE TYPE: Nucleotide with corresponding peptide

SEQUENCE LENGTH: 668 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACG GAA TTG GAT GGG GTG CGG CTA CAC AGG      39
Glu Phe Phe Thr Glu Leu Asp Gly Val Arg Leu His Arg
                 5                  10

TAC GCT CCG GCG TGC AAG CCT CTC CTA CGG GAT GAG GTC      78
Tyr Ala Pro Ala Cys Lys Pro Leu Leu Arg Asp Glu Val
     15                  20                  25

ACA TTC CAG GTC GGG CTC AAC CAA TTC CCG GTT GGG TCA     117
Thr Phe Gln Val Gly Leu Asn Gln Phe Pro Val Gly Ser
             30                  35

CAG CTC CCA TGT GAA CCC GAA CCG GAT GTA ATG GTG GTC     156
Gln Leu Pro Cys Glu Pro Glu Pro Asp Val Met Val Val
 40                  45                  50

ACC TCT ATG CTC ACC GAC CCC TCC CAT ATT ACA GCA GAG     195
Thr Ser Met Leu Thr Asp Pro Ser His Ile Thr Ala Glu
         55                  60                  65

ACG GCT AAG CGT AGG CTG GCC AGA GGG TCT CCC CCT TCT     234
Thr Ala Lys Arg Arg Leu Ala Arg Gly Ser Pro Pro Ser
                 70                  75

TTG GCC AGC TCT TCA GCT AGT CAG TTG TCT GCG CCC TCC     273
Leu Ala Ser Ser Ser Ala Ser Gln Leu Ser Ala Pro Ser
     80                  85                  90

TTG AAG GCG ACA TGC ACC ACC CGT CAT GAC TCC CCG GAC     312
Leu Lys Ala Thr Cys Thr Thr Arg His Asp Ser Pro Asp
             95                 100
```

```
GCT GAC CTC ATA GAG GCC AAC CTC CTG TGG CGG CAG GAG      351
Ala Asp Leu Ile Glu Ala Asn Leu Leu Trp Arg Gln Glu
105                 110                 115

ATG GGC GGG AAC ATC ACC CGT GTG GAG TCA GAG AAT AAG      390
Met Gly Gly Asn Ile Thr Arg Val Glu Ser Glu Asn Lys
    120                 125                 130

GTA GTG ATT TTG GAC TCT TTT GAA CCG CTT CGG GTG GAG      429
Val Val Ile Leu Asp Ser Phe Glu Pro Leu Arg Val Glu
                135                 140

GAG GAT GAG AGG GAA GTA TCC GTA GCG GCG GAT TTC AGT      468
Glu Asp Glu Arg Glu Val Ser Val Ala Ala Asp Phe Ser
    145                 150                 155

GAC TTG AAT GCA GAA TGAATCCCGT GGCTCACTTC                503
Asp Leu Asn Ala Glu
            160

CTAGACTATT TGCCAAAGAA GATGTTGCCC TGGCCATGAT              543

CAAGATGACA CAAACGGTGG CCTTTTGCAG GGAGAACCGC              583

CGTGGAGGCC TGTGTCTGTG GCACTGGTAG CTTCTCTCTG              623

CAGGCAAAGA CCCCATGGCT TAGTTCTTCA TCAGAGTGAG AATTC        668
```

SEQ ID NO.: 20

SEQUENCE TYPE: Nucleotide with corresponding peptide

SEQUENCE LENGTH: 479 base pairs

STRANDNESS: single

TOPOLOGY: linear

MOLECULE TYPE: cDNA

ORIGINAL SOURCE

ORGANISM: human

PROPERTIES: blood-borne non-A, non-B hepatitis specific antigenic protein coding gene

```
GAA TTC TTC ACG GAG TTG GAT GGG GTA CGG CTG CAC AGG      39
Glu Phe Phe Thr Glu Leu Asp Gly Val Arg Leu His Arg
                 5                  10

TAC GCT CCG GCG TGC AAG CCA CTC CTA CGG GAT GAG GTC      78
Tyr Ala Pro Ala Cys Lys Pro Leu Leu Arg Asp Glu Val
     15                  20                  25

ACA TTC CAG GTC GGG CTC AAC CAA TTT CCA GTT GGA TCA     117
Thr Phe Gln Val Gly Leu Asn Gln Phe Pro Val Gly Ser
             30                  35

CAG CTC CCA TGT GAG CCC GAG CCG GAT GTA GCG GTG CTC     156
Gln Leu Pro Cys Glu Pro Glu Pro Asp Val Ala Val Leu
 40                  45                  50

ACT TCC ATG CTC ACC GAC CCC TCC CAC ATT ACA GCA GAG     195
Thr Ser Met Leu Thr Asp Pro Ser His Ile Thr Ala Glu
         55                  60                  65

ACG GCT AAG CGT AGG CTG GCC AGG GGG TCC CCC CCC TCC     234
Thr Ala Lys Arg Arg Leu Ala Arg Gly Ser Pro Pro Ser
                 70                  75

TTG GCC AGC TCT TCA GCT AGT CAG TTG TCT GCG CCC TCC     273
Leu Ala Ser Ser Ser Ala Ser Gln Leu Ser Ala Pro Ser
     80                  85                  90

TTG AAG GCG ACA TGC ACT ACC CAC CAT GAC TCC CCG GAC     312
Leu Lys Ala Thr Cys Thr Thr His His Asp Ser Pro Asp
             95                 100
```

```
GCT GAC CTC ATC GAG GCC AAC CTC CTG TGG CGG CAG GAG      351
Ala Asp Leu Ile Glu Ala Asn Leu Leu Trp Arg Gln Glu
105                 110                 115

ATG GGA GGA AAC ATC ACC CGC GTG GAG TCA GAG AAT AAG      390
Met Gly Gly Asn Ile Thr Arg Val Glu Ser Glu Asn Lys
        120                 125                 130

GTA GTA ATT CTA GAC TCT TTT GAC CCG CTC CGA GCG GAG      429
Val Val Ile Leu Asp Ser Phe Asp Pro Leu Arg Ala Glu
                135                 140

GAG GAT GAG AGG GAA GTG TCC GTT GCG GCG GAG ATC CTG      468
Glu Asp Glu Arg Glu Val Ser Val Ala Ala Glu Ile Leu
    145                 150                 155

CGG AAG ACC AG                                           479
Arg Lys Thr
```

# Fig.1

```
        10         20         30         40         50         60
GAATTCTTCA CGGAATTGGA TGGGGTGCGG CTACACAGGT ACGCTCCGGC GTGCAAGCCT

        70         80         90        100        110        120
CTCCTACGGG ATGAGGTCAC ATTCCAGGTC GGGCTCAACC AATTCCCGGT TGGGTCACAG

       130        140        150        160        170        180
CTCCCATGTG AACCCGAACC GGATGTAATG GTGGTCACCT CTATGCTCAC CGACCCCTCC

       190        200        210        220        230        240
CATATTACAG CAGAGACGGC TAAGCGTAGG CTGGCCAGAG GGTCTCCCCC TTCTTTGGCC

       250        260        270        280        290        300
AGCTCTTCAG CTAGTCAGTT GTCTGCGCCC TCCTTGAAGG CGACATGCAC CACCCGTCAT

       310        320        330        340        350        360
GACTCCCCGG ACGCTGACCT CATAGAGGCC AACCTCCTGT GGCGGCAGGA GATGGGCGGG

       370        380        390        400        410        420
AACATCACCC GTGTGGAGTC AGAGAATAAG GTAGTGATTT TGGACTCTTT TGAACCGCTT

       430        440        450        460        470        480
CGGGTGGAGG AGGATGAGAG GGAAGTATCC GTAGCGGCGG ATTTCAGTGA CTTGAATGCA

       490        500        510        520        530        540
GAATGAATCC CGTGGCTCAC TTCCTAGACT ATTTGCCAAA GAAGATGTTG CCCTGGCCAT

       550        560        570        580        590        600
GATCAAGATG ACACAAACGG TGGCCTTTTG CAGGGAGAAC CGCCGTGGAG GCCTGTGTCT

       610        620        630        640        650        660
GTGGCACTGG TAGCTTCTCT CTGCAGGCAA AGACCCCATG GCTTAGTTCT TCATCAGAGT

       670
GAGAATTC
```

# Fig.2

```
        10         20         30         40         50         60
GAATTCTTCA CGGAGTTGGA TGGGGTACGG CTGCACAGGT ACGCTCCGGC GTGCAAGCCA

        70         80         90        100        110        120
CTCCTACGGG ATGAGGTCAC ATTCCAGGTC GGGCTCAACC AATTTCCAGT TGGATCACAG

       130        140        150        160        170        180
CTCCCATGTG AGCCCGAGCC GGATGTAGCG GTGCTCACTT CCATGCTCAC CGACCCCTCC

       190        200        210        220        230        240
CACATTACAG CAGAGACGGC TAAGCGTAGG CTGGCCAGGG GGTCCCCCCC CTCCTTGGCC

       250        260        270        280        290        300
AGCTCTTCAG CTAGTCAGTT GTCTGCGCCC TCCTTGAAGG CGACATGCAC TACCCACCAT

       310        320        330        340        350        360
GACTCCCCGG ACGCTGACCT CATCGAGGCC AACCTCCTGT GGCGGCAGGA GATGGGAGGA

       370        380        390        400        410        420
AACATCACCC GCGTGGAGTC AGAGAATAAG GTAGTAATTC TAGACTCTTT TGACCCGCTC

       430        440        450        460        470        480
CGAGCGGAGG AGGATGAGAG GGAAGTGTCC GTTGCGGCGG AGATCCTGCG GAAGACCAG
```

# Fig.3

```
          10          20          30          40
TCA CTC AAT CCT CGA CGG TGC TGC CGG TGC GGC AAT CCG GAA CGA TAC
Ser Leu Asn Pro Arg Arg Cys Cys Arg Cys Gly Asn Pro Glu Arg Tyr

 50         60          70          80          90
CGA CGC CGG ATC GCC CTG CTG CCC CCA CGC ATT TAC CGC CCG GAC TGT
Arg Arg Arg Ile Ala Leu Leu Pro Pro Arg Ile Tyr Arg Pro Asp Cys

    100         110         120         130         140
CAG CCT GTA GTT CCC CAG CGC CAG TTG CGT GAA GCG GTA TGT GGT TTC
Gln Pro Val Val Pro Gln Arg Gln Leu Arg Glu Ala Val Cys Gly Phe

     150         160         170         180         190
CGT CGT CCG GGC CGT GCT GAC CAG CCG CTC ACT GCC GTC GTC CGT GTT
Arg Arg Pro Gly Arg Ala Asp Gln Pro Leu Thr Ala Val Val Arg Val

        200         210         220         230         240
ACG GTC AGA CGG AGC AGG AAA CTC ACG CCT TCA CAC TTC GGT GTG TCC
Thr Val Arg Arg Ser Arg Lys Leu Thr Pro Ser His Phe Gly Val Ser

          250        260        270        280        290
CAT CGC GCC AGC ACC TGATATTCCC CGCTGTCTGC AGTGACTTCT GCGGTCAGGT
His Arg Ala Ser Thr

    300        310        320        330        340
GCTGCACCGC TCGTGACACC ATTCACCGTG CCACTCTGTT CGCCGTCAAA

    350        360        370        380        390
GTGCGCCCCG TTATCCACGA TGGCCTCTTT TTCCGGCACA TGCTGCACGG

    400        410        420        430        440
CGGTGATGGC ATACGTGCCG TCGTCGTTCT CACGGATACTC ACGCAGCGG

    450        460        470        480        490
AACAGTCCTG GCGCAGCGTC GGCAGCTTCA GCTCCCATAC GCTGTATTCA GCT
```

# Fig.4(1)

```
         10          20          30          40
GAA TTC TTC ACA GAG TTG GAC GGG GTG CGG CTG CAC AGG TAC GCT CCG
Glu Phe Phe Thr Glu Leu Asp Gly Val Arg Leu His Arg Tyr Ala Pro

 50          60          70          80          90
GCG TGC AGA CCT CTC CTG CGG GAT GAG GTC ACA TTC CAG GTC GGG CTC
Ala Cys Arg Pro Leu Leu Arg Asp Glu Val Thr Phe Gln Val Gly Leu

    100         110         120         130         140
AAC CAA TAC CCG GTT GGG TCA CCG CTC CCA TGT GAG CCC GAA CCG GAT
Asn Gln Tyr Pro Val Gly Ser Pro Leu Pro Cys Glu Pro Glu Pro Asp

     150         160         170         180         190
GTA ACA GTG GTC ACT TCC ATG CTC ACC GAC CCC TCC CAC ATT ACA GCG
Val Thr Val Val Thr Ser Met Leu Thr Asp Pro Ser His Ile Thr Ala

        200         210         220         230         240
GAA ACT GCC AGG CGT AGG TTG GCC AGG GGG AGT CCC CCT TCC TTG GCC
Glu Thr Ala Arg Arg Arg Leu Ala Arg Gly Ser Pro Pro Ser Leu Ala

         250         260         270         280
AGC TCT TCA GCT AGT CAG TTG TCT GCA CCT TCC TTG AAG GCG ACA TGT
Ser Ser Ser Ala Ser Gln Leu Ser Ala Pro Ser Leu Lys Ala Thr Cys

 290         300         310         320         330
ACT ACC CAT CAT GAC TCT CCA GAC GCT GAT CTC ATC GAG GCC AAC CTT
Thr Thr His His Asp Ser Pro Asp Ala Asp Leu Ile Glu Ala Asn Leu

    340         350         360         370         380
CTA TGG CGG CAG GAG ATG GGC GGG AAC ATC ACC CGT GTG GAG TCA GAG
Leu Trp Arg Gln Glu Met Gly Gly Asn Ile Thr Arg Val Glu Ser Glu

     390         400         410         420         430
AAT AAG GTA GTA ATC CTA GAC TCT TTT GAC CCG CTT CGA GCG GAG GAG
Asn Lys Val Val Ile Leu Asp Ser Phe Asp Pro Leu Arg Ala Glu Glu

        440         450         460         470         480
GAT GAG AGG GAA GTA TCC GTT GCG GCG GAG ATC CTG CGG AGA ACC AGG
Asp Glu Arg Glu Val Ser Val Ala Ala Glu Ile Leu Arg Arg Thr Arg

         490         500         510         520
AGA TTC CCC CCG GCG ATA CCT GTA TGG GCG CGC CCG GAC TAC AAC CCG
Arg Phe Pro Pro Ala Ile Pro Val Trp Ala Arg Pro Asp Tyr Asn Pro

 530         540         550         560         570
CCA CTG ATA GAA TCT TGG AAG GAC CCA GAC TAC GTC CCA CCG GTG GTA
Pro Leu Ile Glu Ser Trp Lys Asp Pro Asp Tyr Val Pro Pro Val Val

    580         590         600         610         620
CAC GGG TGT CCA TTG CCA CCT GCC AAG ACC CCT CAA GTG GAT ATT CAG
His Gly Cys Pro Leu Pro Pro Ala Lys Thr Pro Gln Val Asp Ile Gln

     630         640         650         660         670
ACC TCT TTG AGG CTT TCG TTG GAA ACG GGA TTT CTT CAT ACT ATG CTA
Thr Ser Leu Arg Leu Ser Leu Glu Thr Gly Phe Leu His Thr Met Leu
```

Fig.4(2)

```
        680
GAC AGA AGA ATT C
Asp Arg Arg Ile
```

# Fig.5

```
             10          20          30          40
TG ATA GCG TTC GCT TCG CGG GGA AAC CAC GTC TCC CCC ACG CAC TAT
   Ile Ala Phe Ala Ser Arg Gly Asn His Val Ser Pro Thr His Tyr

  50         60          70          80          90
GTG CCT GAA AGC GAC GCT GCA GCG CGT GTC ACC CAG ATC CTC TCC AGC
Val Pro Glu Ser Asp Ala Ala Ala Arg Val Thr Gln Ile Leu Ser Ser

    100         110         120         130         140
CTT ACC ATC ACT CAG CTG TTG AAG AGG CTC CAC CAG TGG ATC AAT GAG
Leu Thr Ile Thr Gln Leu Leu Lys Arg Leu His Gln Trp Ile Asn Glu

       150         160         170         180         190
GAC TGC TCC ACG CCA TGC TCC GGT TCG TGG CTT AGG GAT GTT TGG GAC
Asp Cys Ser Thr Pro Cys Ser Gly Ser Trp Leu Arg Asp Val Trp Asp

         200         210         220         230
TGG ATA TGC ACG GTG TTG ACT GAC TTC AAA ACC TGG CTC CAG TCC AAG
Trp Ile Cys Thr Val Leu Thr Asp Phe Lys Thr Trp Leu Gln Ser Lys

240         250         260         270         280
CTC CTG CCG CGA TTG CCG GGA GTC CCT TTC CTT TCA TGC CAA CGA GGG
Leu Leu Pro Arg Leu Pro Gly Val Pro Phe Leu Ser Cys Gln Arg Gly

  290        300         310         320         330
TAC AAG GGA GTC TGG CGG GGA GAT GGT GTC ATG CAA ACC ACC TGC CCA
Tyr Lys Gly Val Trp Arg Gly Asp Gly Val Met Gln Thr Thr Cys Pro

    340         350         360         370         380
TGT GGA GCA CAG ATC AGT GGG CAT GTC AAA AAT GGC TCC ATG AGG ATC
Cys Gly Ala Gln Ile Ser Gly His Val Lys Asn Gly Ser Met Arg Ile

       390         400         410         420         430
GTT GGG CCT AGA ACC TGT AGC AAC ACG TGG CAC GGA ACG TTC CCT ATC
Val Gly Pro Arg Thr Cys Ser Asn Thr Trp His Gly Thr Phe Pro Ile

         440         450         460         470
AAC GCG TAC ACC ACA GGC CCC TGC ACA CCC TCC CCG GCG CCC AAC TAC
Asn Ala Tyr Thr Thr Gly Pro Cys Thr Pro Ser Pro Ala Pro Asn Tyr

480         490         500         510         520
TCT AGG GCG TTG TGG CGG GTG GCT GCT GAG GAG TAC GTG GAG GTC ACG
Ser Arg Ala Leu Trp Arg Val Ala Ala Glu Glu Tyr Val Glu Val Thr

  530        540         550         560         570
CGG GTG GGG GAT TTC CAC TAC GTG ACG GGC ATG ACC ACT GAC AAC GTA
Arg Val Gly Asp Phe His Tyr Val Thr Gly Met Thr Thr Asp Asn Val

    580         590         600
AGA TGC CCA TGC CAG GTT CCG GCC CCC GAA TTC
Arg Cys Pro Cys Gln Val Pro Ala Pro Glu Phe
```

# Fig.6

```
                10                  20                  30                  40
GAA TTC TTC ACA GAG TTG GAT GGG GTG CGG TTG CAC AGG TAC GCT CCG
Glu Phe Phe Thr Glu Leu Asp Gly Val Arg Leu His Arg Tyr Ala Pro

 50                 60                  70                  80                  90
GCT GCA AAG CCT CTC CTA CGG GAT GAG GTC ACA TTT CAG GTC GGG CTC
Ala Ala Lys Pro Leu Leu Arg Asp Glu Val Thr Phe Gln Val Gly Leu

    100                 110                 120                 130                 140
AAC CAA TTC CCG GTT GGG TCA CAG CTC CCA TGT GAG CCC GAA CCG GAT
Asn Gln Phe Pro Val Gly Ser Gln Leu Pro Cys Glu Pro Glu Pro Asp

      150                 160                 170                 180                 190
GTA ACA GTG ATC ACC TCC ATG CTC ACC GAC CCC TCC CAC ATT ACA GCA
Val Thr Val Ile Thr Ser Met Leu Thr Asp Pro Ser His Ile Thr Ala

          200                 210                 220                 230                 240
GAG GCG GCT GGG CGT AGG CTG GCC AGA GGG TCT CCC CCT TCC TTG GCC
Glu Ala Ala Gly Arg Arg Leu Ala Arg Gly Ser Pro Pro Ser Leu Ala

                250                 260                 270                 280
AGC TCT TCG GCT AGT CAG TTG TCT GCG CCC TTC CCT GTT GAA GGC CGA
Ser Ser Ser Ala Ser Gln Leu Ser Ala Pro Phe Pro Val Glu Gly Arg

 290                 300                 310                 320                 330
CAT GCA CTA CCC GTC ATG ACT CCC CAG ACG CTG ACC TCA TCG AGG CCA
His Ala Leu Pro Val Met Thr Pro Gln Thr Leu Thr Ser Ser Arg Pro

    340                 350                 360                 370                 380
ATC TCC TGT GGC GGC AGG AGA TGG GAG GGA ACA TCA CCC GCG TGG AGT
Ile Ser Cys Gly Gly Arg Arg Trp Glu Gly Thr Ser Pro Ala Trp Ser

      390                 400             410             420
CAG AGA ACA AGG TAC TAATCCTAGA CTCTTTTGAC CCGCTCCGAG
Gln Arg Thr Arg Tyr

430            440            450            460            470
CGGAGGAGGA TGAGAGGGAG ATATCTGTTG CGGCCCAGCT GAGC
```

# Fig.7

```
             10                  20                  30                  40
GAA TTC TTC ACA GAG CTG GAT GGG GTG CGG TTG CAC AGG TAC GCT CCG
Glu Phe Phe Thr Glu Leu Asp Gly Val Arg Leu His Arg Tyr Ala Pro

 50                 60                  70                 80                 90
GCG TGC AAG CCT CTC CTA CGG GAT GAG GTC ACA TTT CAG GTC GGG CTC
Ala Cys Lys Pro Leu Leu Arg Asp Glu Val Thr Phe Gln Val Gly Leu

    100                 110                120                 130                140
AAC CAA TTC CCG GTT GGG TCA CAG CTC CCG TGT GAG CCC GAA CCG GAT
Asn Gln Phe Pro Val Gly Ser Gln Leu Pro Cys Glu Pro Glu Pro Asp

      150                 160                170                 180                190
GTA ACG GTG ATC ACT TCC ATG CTC ACC GAC CCC TCC CAC ATT ACA GCA
Val Thr Val Ile Thr Ser Met Leu Thr Asp Pro Ser His Ile Thr Ala

         200                 210                 220                 230                240
GAG ACG GCT GGG CGT AGG CTG GCC AGA GGG TCT CCC CCT TCC TTG GCC
Glu Thr Ala Gly Arg Arg Leu Ala Arg Gly Ser Pro Pro Ser Leu Ala

             250                 260                 270                 280
AGC TCT TCG GCT AGT CAG TTG TCT GCG CCC TCC TTG AAG GCA ACA TGC
Ser Ser Ser Ala Ser Gln Leu Ser Ala Pro Ser Leu Lys Ala Thr Cys

 290                300                 310                 320                330
ACT ACC CGT CAT GAC TCC CCA GAC GCT GAC CTC ATC GAG GCC AAT CTC
Thr Thr Arg His Asp Ser Pro Asp Ala Asp Leu Ile Glu Ala Asn Leu

    340                 350                360                 370                380
CTG TGG CGG CAG GAG ATG GGA GGG AAC ATC ACC CGC GTG GAG TCA GAG
Leu Trp Arg Gln Glu Met Gly Gly Asn Ile Thr Arg Val Glu Ser Glu

      390                 400                410                 420                430
AAC AAG GTA GTA ATC CTA GAC TCT TTT GAC CCG CTC CGA GCG GAG GAG
Asn Lys Val Val Ile Leu Asp Ser Phe Asp Pro Leu Arg Ala Glu Glu

         440                 450                 460                 470                480
GAT GAG AGG GAG ATA TCT GTT GCG GCG GAG ATC CTA CGG AAA TCT AGG
Asp Glu Arg Glu Ile Ser Val Ala Ala Glu Ile Leu Arg Lys Ser Arg

             490                 500                 510                 520
AAA TTC CCC CCA GCA TTA CCC ATA TGG GCG CGC CCG GAC TAC AACC
Lys Phe Pro Pro Ala Leu Pro Ile Trp Ala Arg Pro Asp Tyr Asn
```

# Fig.8

```
                10          20          30          40
GAA TTC TTC ACA GAG TTG GAT GGG GTG CGG CTG CAC AGG TAC GCT CCG
Glu Phe Phe Thr Glu Leu Asp Gly Val Arg Leu His Arg Tyr Ala Pro

 50          60          70          80          90
GCG TGC AAA CCT CTC CTG CGG GAT GAG GTC ACG TTC CAG GTC GGG CTC
Ala Cys Lys Pro Leu Leu Arg Asp Glu Val Thr Phe Gln Val Gly Leu

    100         110         120         130         140
AAC CAA TAC CCG GTT GGA TCA CAG CTC CCA TGC GAG CCC GAA CCG GAT
Asn Gln Tyr Pro Val Gly Ser Gln Leu Pro Cys Glu Pro Glu Pro Asp

       150         160         170         180         190
GTG GCG GTG CTC ACT TCC ATG CTC ACC GAC CCC ACC CAC ATT ACA GCA
Val Ala Val Leu Thr Ser Met Leu Thr Asp Pro Thr His Ile Thr Ala

           200         210         220         230         240
GAA GCG GCT AGG CGC AGG CTG GCC AGA GGG TCT CCT CCT TCC TTG GCC
Glu Ala Ala Arg Arg Arg Leu Ala Arg Gly Ser Pro Pro Ser Leu Ala

               250         260         270         280
AGC TCT TCA GCT AGT CAG TTG TCT GCG CCC TCC TTG AAG GCG ACA TGC
Ser Ser Ser Ala Ser Gln Leu Ser Ala Pro Ser Leu Lys Ala Thr Cys

 290         300         310         320         330
ACT ACC CAT CAT GAC TCC CCA GAC GCT GAC CTC ATC GAG GCC AAC CTC
Thr Thr His His Asp Ser Pro Asp Ala Asp Leu Ile Glu Ala Asn Leu

    340         350         360         370         380
CTG TGG CGG CAG GAG ATG GGC GGA AAC ATC ACC CGC GTG GAG TCA GAG
Leu Trp Arg Gln Glu Met Gly Gly Asn Ile Thr Arg Val Glu Ser Glu

       390         400         410         420         430
AAT AAG GTA GTA ATT CTA GAC TCT TTT GAA CCG CTT CGA GCG GAA GAG
Asn Lys Val Val Ile Leu Asp Ser Phe Glu Pro Leu Arg Ala Glu Glu

           440         450         460         470         480
GAT GAG AGG GAA GTA TCC GTT GCG GCA GAG ATC CTG CGG AAA ACC AGG
Asp Glu Arg Glu Val Ser Val Ala Ala Glu Ile Leu Arg Lys Thr Arg

               490         500         510         520
AGA TTC CCC GCA GCG ATG CCC ATA TGG GCA CGT CCG GAC TAC AAC CCA
Arg Phe Pro Ala Ala Met Pro Ile Trp Ala Arg Pro Asp Tyr Asn Pro

 530         540         550         560         570
CCA TTA CTA CAG TCC TGG AAG GAC CCG GAC TAC GTC CCT CCG GTG GTG
Pro Leu Leu Gln Ser Trp Lys Asp Pro Asp Tyr Val Pro Pro Val Val

    580         590
CAC GGG TGC CCA TTG CCA CCT GC
His Gly Cys Pro Leu Pro Pro
```

# Fig.9(1)

```
           10                  20                  30                  40
GAA TTC TTC ACA GAG TTG GAT GGG GTG CGG CTG CAC AGG TAC GCT CCG
Glu Phe Phe Thr Glu Leu Asp Gly Val Arg Leu His Arg Tyr Ala Pro

 50                 60                  70                  80                  90
GCG TGC AAA CCT CTC CTG CGG GAT GAG GTC ACG TTC CAG GTC GGG CTC
Ala Cys Lys Pro Leu Leu Arg Asp Glu Val Thr Phe Gln Val Gly Leu

    100                110                 120                 130                 140
AAC CAA TAC CCG GTT GGA TCA CAG CTC CCA TGC GAG CCC GAA CCG GAT
Asn Gln Tyr Pro Val Gly Ser Gln Leu Pro Cys Glu Pro Glu Pro Asp

    150                 160                 170                 180                 190
GTG GCG GTG CTC ACT TCC ATG CTC ACC GAC CCC ACC CAC ATT ACA GCA
Val Ala Val Leu Thr Ser Met Leu Thr Asp Pro Thr His Ile Thr Ala

        200                 210                 220                 230                 240
GAA GCG GCT AGG CGC AGG CTG GCC AGA GGG TCT CCT CCT TCC TTG GCC
Glu Ala Ala Arg Arg Arg Leu Ala Arg Gly Ser Pro Pro Ser Leu Ala

           250                 260                 270                 280
AGC TCT TCA GCT AGT CAG TTG TCT GCG CCC TCC TTG AAG GCG ACA TGC
Ser Ser Ser Ala Ser Gln Leu Ser Ala Pro Ser Leu Lys Ala Thr Cys

 290                300                 310                 320                 330
ACT ACC CAT CAT GAC TCC CCA GAC GCT GAC CTC ATC GAG GCC AAC CTC
Thr Thr His His Asp Ser Pro Asp Ala Asp Leu Ile Glu Ala Asn Leu

    340                 350                 360                 370                 380
CTG TGG CGG CAG GAG ATG GGC GGA AAC ATC ACC CGC GTG GAG TCA GAG
Leu Trp Arg Gln Glu Met Gly Gly Asn Ile Thr Arg Val Glu Ser Glu

    390                 400                 410                 420                 430
AAT AAG GTA GTA ATT CTA GAC TCT TTT GAA CCG CTT CGA GCG GAA GAG
Asn Lys Val Val Ile Leu Asp Ser Phe Glu Pro Leu Arg Ala Glu Glu

        440                 450                 460                 470                 480
GAT GAG AGG GAA GTA TCC GTT GCG GCA GAG ATC CTG CGG AAA ACC AGG
Asp Glu Arg Glu Val Ser Val Ala Ala Glu Ile Leu Arg Lys Thr Arg

           490                 500                 510                 520
AGA TTC CCC GCA GCG ATG CCC ATA TGG GCA CGT CCG GAC TAC AAC CCA
Arg Phe Pro Ala Ala Met Pro Ile Trp Ala Arg Pro Asp Tyr Asn Pro

 530                540                 550                 560                 570
CCA TTA CTA CAG TCC TGG AAG GAC CCG GAC TAC GTC CCT CCG GTG GTG
Pro Leu Leu Gln Ser Trp Lys Asp Pro Asp Tyr Val Pro Pro Val Val

    580                 590                 600                 610                 620
CAC GGG TGC CCA TTG CCA CCT GCC AAG GCC CCT CCA GTA CCA CCT CCA
His Gly Cys Pro Leu Pro Pro Ala Lys Ala Pro Pro Val Pro Pro Pro

    630                 640                 650                 660                 670
AGG AGA AAG AGG ACG GTT GTC CTG ACA GAA TCC ACC GTG TCT TCC GCC
Arg Arg Lys Arg Thr Val Val Leu Thr Glu Ser Thr Val Ser Ser Ala
```

# Fig.9(2)

```
        680         690         700         710         720
TTG GCG GAG CTT GCT ACA AAG ACC TTC GGC GGG TCC GGA TCA TCG GCC
Leu Ala Glu Leu Ala Thr Lys Thr Phe Gly Gly Ser Gly Ser Ser Ala

            730         740         750         760
GCC GAC AGC GGC ACA GCA AGC GGC CCT CCT GGC CAG GCC TCC GAC GAT
Ala Asp Ser Gly Thr Ala Ser Gly Pro Pro Gly Gln Ala Ser Asp Asp

 770         780         790         800         810
GGA GAT ACA GGA TCC GAC GTT GAG TCG TAC TCC TCC ATG CCC CCC CTT
Gly Asp Thr Gly Ser Asp Val Glu Ser Tyr Ser Ser Met Pro Pro Leu

    820         830         840         850         860
GAG GGA GAG CCG GGG GAC CCC GAC CTC AGC GAC GGG TCT TGG TCT ACT
Glu Gly Glu Pro Gly Asp Pro Asp Leu Ser Asp Gly Ser Trp Ser Thr

     870         880         890         900         910
GTG AGT GAG GAG GCT GGT GAG GAT GTC GTC TGC TGC TCG ATG TCC TAC
Val Ser Glu Glu Ala Gly Glu Asp Val Val Cys Cys Ser Met Ser Tyr

        920         930         940         950         960
ACA TGG ACA GGC GCC TTA ATC ACA CCA TGC ACC GCG GAG GAG AGC AAG
Thr Trp Thr Gly Ala Leu Ile Thr Pro Cys Thr Ala Glu Glu Ser Lys

            970         980         990         1000
CTG CCC ATC AAC CCG TTG AGC AAC TCT TTG CTG CGG GCA TCT GCT CGG
Leu Pro Ile Asn Pro Leu Ser Asn Ser Leu Leu Arg Ala Ser Ala Arg

 1010        1020        1030        1040        1050
GCG TAT CAT CAA CTG ATG AGC AAG AAG GAT ATA ATT CCT ACG CCC TCT
Ala Tyr His Gln Leu Met Ser Lys Lys Asp Ile Ile Pro Thr Pro Ser

    1060        1070        1080        1090        1100
CAG CCG ATG AAC AGT TGG AAT AGG TTG TTA GCG GTA ACT AAG ATT AGT
Gln Pro Met Asn Ser Trp Asn Arg Leu Leu Ala Val Thr Lys Ile Ser

     1110        1120        1130        1140        1150
ATG GTA ATT AGG AAA ATG AGT AGA TAT TTG AAG AAC TGATTAATGT
Met Val Ile Arg Lys Met Ser Arg Tyr Leu Lys Asn

        1160        1170        1180
TTGGGTCTGA GTTTATATAT CACAGTGAGA ATTC
```

# Fig.10

```
             10                  20                  30                  40
GGG CAT GTC AAA AAT GGC TCC ATG AGG ATC GTT GGG CCT AGA ACC TGT
Gly His Val Lys Asn Gly Ser Met Arg Ile Val Gly Pro Arg Thr Cys

 50                60                  70                80                  90
AGC AAC ACG TGG CAC GGA ACG TTC CCT ATC AAC GCG TAC ACC ACA GGC
Ser Asn Thr Trp His Gly Thr Phe Pro Ile Asn Ala Tyr Thr Thr Gly

    100                 110                 120                 130                 140
CCC TGC ACA CCC TCC CCG GCG CCC AAC TAC TCT AGG GCG TTG TGG CGG
Pro Cys Thr Pro Ser Pro Ala Pro Asn Tyr Ser Arg Ala Leu Trp Arg

       150                 160                 170                 180                 190
GTG GCT GCT GAG GAG TAC GTG GAG GTC ACG CGG GTG GGG GAT TTC CAC
Val Ala Ala Glu Glu Tyr Val Glu Val Thr Arg Val Gly Asp Phe His

          200                 210                 220                 230                 240
TAC GTG ACG GGC ATG ACC ACT GAC AAC GTA AGA TGC CCA TGC CAG GTT
Tyr Val Thr Gly Met Thr Thr Asp Asn Val Arg Cys Pro Cys Gln Val

             250
CCG GCC CCC GAA TTC
Pro Ala Pro Glu Phe
```

# Fig.11

```
                 10                  20                  30                  40
GAA TTC TTC ACA GAG TTG GAT GGG GTG CGG TTG CAC AGG TAC GCT CCG
Glu Phe Phe Thr Glu Leu Asp Gly Val Arg Leu His Arg Tyr Ala Pro

 50                  60                  70                  80                  90
GCG TGC AAA CCT CTC CTA CGG GAT GAG GTC ACA TTT CAG GTC GGG CTC
Ala Cys Lys Pro Leu Leu Arg Asp Glu Val Thr Phe Gln Val Gly Leu

    100                 110                 120                 130                 140
AAC CAA TTC CCG GTT GGG TCA CAG CTC CCA TGT GAG CCC GAA CCG GAT
Asn Gln Phe Pro Val Gly Ser Gln Leu Pro Cys Glu Pro Glu Pro Asp

      150                 160                 170                 180                 190
GTA ACA GTG ATC ACC TCC ATG CTC ACC GAC CCC TCC CAC ATT ACA GCA
Val Thr Val Ile Thr Ser Met Leu Thr Asp Pro Ser His Ile Thr Ala

         200                 210                 220                 230                 240
GAG ACG GCT GGG CGT AGG CTG GCC AGA GGG TCT CCC CCT TCC TTG GCC
Glu Thr Ala Gly Arg Arg Leu Ala Arg Gly Ser Pro Pro Ser Leu Ala

                 250                 260                 270                 280
AGC TCT TCG GCT AGT CAG TTG TCT GCG CCT TCC TTG AAG GCG ACA TGC
Ser Ser Ser Ala Ser Gln Leu Ser Ala Pro Ser Leu Lys Ala Thr Cys

 290                 300                 310                 320                 330
ACT ACC CGT CAT GAC TCC CCA GAC GCT GAC CTC ATC GAG GCC AAT CTC
Thr Thr Arg His Asp Ser Pro Asp Ala Asp Leu Ile Glu Ala Asn Leu

    340                 350                 360                 370                 380
CTG TGG CGG CAG GAG ATG GGA GGG AAC ATC ACC CGC GTG GAG TCA GAG
Leu Trp Arg Gln Glu Met Gly Gly Asn Ile Thr Arg Val Glu Ser Glu

      390                 400                 416                 420                 430
AAC AAG GTA GTA ATC CTA GAC TCT TTT GAC CCG CTC CGA GCG GAG GAG
Asn Lys Val Val Ile Leu Asp Ser Phe Asp Pro Leu Arg Ala Glu Glu

         440                 450                 460                 470                 480
GAT GAG AGG GAG ATA TCT GTT GCG GCG GAG ATC CTA CGG AAA TCT AGG
Asp Glu Arg Glu Ile Ser Val Ala Ala Glu Ile Leu Arg Lys Ser Arg

                 490                 500                 510                 520
AAA TTC CCC CCA GCA TTA CCC ATA TGG GCG CGC CCG GAC TAC AAC CCA
Lys Phe Pro Pro Ala Leu Pro Ile Trp Ala Arg Pro Asp Tyr Asn Pro

 530                 540                 550
CCA CTG CTA GAG TCT TGG CCA GCT G
Pro Leu Leu Glu Ser Trp Pro Ala
```

# Fig.12(1)

```
            10          20          30          40
CG GCT TTC GTG GGC GCC GGC ATA GCC GGC GCG GCT GTT GGC AGC ATA
   Ala Phe Val Gly Ala Gly Ile Ala Gly Ala Ala Val Gly Ser Ile

 50          60          70          80          90
GGC CTT GGG AAG GTG CTT GTG GAC ATC CTG GCG GGT TAT GGA GCA GGG
Gly Leu Gly Lys Val Leu Val Asp Ile Leu Ala Gly Tyr Gly Ala Gly

    100         110         120         130         140
GTG GCA GGC GCA CTC GTG GTC TTT AAG GTT ATG AGT GGC GAC ATG CCC
Val Ala Gly Ala Leu Val Val Phe Lys Val Met Ser Gly Asp Met Pro

       150         160         170         180         190
TCC ACC GAG GAC CTG GTC AAC TTA CTC CCT GCC ATC CTT TCC CCT GGC
Ser Thr Glu Asp Leu Val Asn Leu Leu Pro Ala Ile Leu Ser Pro Gly

          200         210         220         230
GCC CTG GTC GTC GGG GTC GTG TGC GCA CAG ATA CTG CGT CGA CAT GTC
Ala Leu Val Val Gly Val Val Cys Ala Gln Ile Leu Arg Arg His Val

240         250         260         270         280
GGC CCA GGG GAG GGA GCT GTG CAG TGG ATG AAC CGG CTG ATA GCG TTC
Gly Pro Gly Glu Gly Ala Val Gln Trp Met Asn Arg Leu Ile Ala Phe

 290         300         310         320         320
GCT TCG CGG GGT AAC CAC GTC TCC CCC ACG CAT TAT GTG CCT GAA AGC
Ala Ser Arg Gly Asn His Val Ser Pro Thr His Tyr Val Pro Glu Ser

    340         350         360         370         380
GAC GCT GCG AGT CGT GTC ACC CAG ATC CTC TCC AGC CTT ACC ATC ACT
Asp Ala Ala Ser Arg Val Thr Gln Ile Leu Ser Ser Leu Thr Ile Thr

       390         400         410         420         430
CAG CTG TTG AAG AGG CTC CAC CAG TGG ATT AAT GAG GAC TGC TCC ACG
Gln Leu Leu Lys Arg Leu His Gln Trp Ile Asn Glu Asp Cys Ser Thr

          440         450         460         470
CCA TGC TCC GGC ACG TGG CTC AGG GAT GTT TGG GAC TGG ATA TGC ACG
Pro Cys Ser Gly Thr Trp Leu Arg Asp Val Trp Asp Trp Ile Cys Thr

480         490         500         510         520
GTG TTG GCT GAC TTC AAG ACC TGG CTC CAG TCC AAG CTC CTG CCG CGG
Val Leu Ala Asp Phe Lys Thr Trp Leu Gln Ser Lys Leu Leu Pro Arg

 530         540         550         560         570
TTA CCG GGG GTC CCT TTC CTC TCA TGT CAG CGT GGG TAC AAG GGA GTT
Leu Pro Gly Val Pro Phe Leu Ser Cys Gln Arg Gly Tyr Lys Gly Val

    580         590         600         610         620
TGG CGG GGA GAT GGC ATC ATG CAC ACC ACC TGC CCA TGC GGA GCC CAA
Trp Arg Gly Asp Gly Ile Met His Thr Thr Cys Pro Cys Gly Ala Gln

       630         640         650         660         670
ATC ACC GGA CAT GTC AAA AAC GGG TCC ATG AGG ATC GCC GGG CCT AAA
Ile Thr Gly His Val Lys Asn Gly Ser Met Arg Ile Ala Gly Pro Lys
```

# Fig.12(2)

```
        680                 690              700              710
ACC TGC AGC AAC ACG TGG CAC GGA ACG TTC CCC ATT AAC GCA TAC ACC
Thr Cys Ser Asn Thr Trp His Gly Thr Phe Pro Ile Asn Ala Tyr Thr

720             730              740              750              760
ACA GGC CCC TGC ACA CCC TCT CCG GCG CCA AAC TAC TCC AGG GCG TTG
Thr Gly Pro Cys Thr Pro Ser Pro Ala Pro Asn Tyr Ser Arg Ala Leu

  770                780              790              800                810
TGG CGG GTG GCT GCG GAG GAG TAC GTG GAG GTC ACG CGG GTG GGG GAT
Trp Arg Val Ala Ala Glu Glu Tyr Val Glu Val Thr Arg Val Gly Asp

    820              830              840              850              860
TTC CAC TAC GTG ACG GGC ATG ACC ACT GAC AAT GTA AAA TGC CCA TGC
Phe His Tyr Val Thr Gly Met Thr Thr Asp Asn Val Lys Cys Pro Cys

        870              880
CAG GTT CCG GCC CCC GAA TTC
Gln Val Pro Ala Pro Glu Phe
```

# Fig.13

```
                10          20          30          40
GAA TTC TTC ACA GAG TTG GAC GGG GTG CGG CTG CAC AGG TAC GCT CCG
Glu Phe Phe Thr Glu Leu Asp Gly Val Arg Leu His Arg Tyr Ala Pro

 50          60          70          80          90
GCG TGC AGA CCT CTC CTG CGG GAT GAG GTC ACA TTC CAG GTC GGG CTC
Ala Cys Arg Pro Leu Leu Arg Asp Glu Val Thr Phe Gln Val Gly Leu

    100         110         120         130         140
AAC CAA TAC CCG GTT GGG TCA CAG CTC CCA TGT GAG CCC GAA CCG GAT
Asn Gln Tyr Pro Val Gly Ser Gln Leu Pro Cys Glu Pro Glu Pro Asp

     150         160         170         180         190
GTA ACA GTG GTC ACT TCC ATG CTC ACC GAC CCC TCC CAC ATT ACA GCG
Val Thr Val Val Thr Ser Met Leu Thr Asp Pro Ser His Ile Thr Ala

        200         210         220         230         240
GAA ACT GCC AGG CGT AGG TTG GCC AGG GGG AGT CCC CCT TCC TTG GCC
Glu Thr Ala Arg Arg Arg Leu Ala Arg Gly Ser Pro Pro Ser Leu Ala

                250         260         270         280
AGC TCT TCA GCT AGT CAG TTG TCT GCA CCT TCC TTG AAG GCG ACA TGT
Ser Ser Ser Ala Ser Gln Leu Ser Ala Pro Ser Leu Lys Ala Thr Cys

 290         300         310         320         330
ACT ACC CAT CAT GAC TCT CCA GAC GCT GAT CTC ATC GAG GCC AAC CTT
Thr Thr His His Asp Ser Pro Asp Ala Asp Leu Ile Glu Ala Asn Leu

    340         350         360         370         380
CTA TGG CGG CAG GAG ATG GGC GGG AAC ATC ACC CGT GTG GAG TCA GAG
Leu Trp Arg Gln Glu Met Gly Gly Asn Ile Thr Arg Val Glu Ser Glu

     390         400         410         420         430
AAT AAG GTA GTA ATC CTA GAC TCT TTT GAC CCG CTT CGA GCG GAG GAG
Asn Lys Val Val Ile Leu Asp Ser Phe Asp Pro Leu Arg Ala Glu Glu

        440         450         460         470         480
GAT GAG AGG GAA GTA TCC GTT GCG GCG GAG ATC CTG CGG AGA ACC AGG
Asp Glu Arg Glu Val Ser Val Ala Ala Glu Ile Leu Arg Arg Thr Arg

                490         500         510         520
AGA TTC CCC CCG GCG ATA CCT GTA TGG GCG CGC CCG GAC CAG CT
Arg Phe Pro Pro Ala Ile Pro Val Trp Ala Arg Pro Asp Gln
```

# Fig.14

```
              10                  20                  30                  40
GAA TTC AGC AAA GTT TCC AGA TAC AAG ATT AAT GGA CAC AAA TCA GTA
Glu Phe Ser Lys Val Ser Arg Tyr Lys Ile Asn Gly His Lys Ser Val

 50               60                  70                  80                  90
GCT CTT CCA TAC ATC AAC AGC TAC CAA GCA GAG AAT CAC ATC AAG AAC
Ala Leu Pro Tyr Ile Asn Ser Tyr Gln Ala Glu Asn His Ile Lys Asn

    100                 110                 120                 130                 140
TCA ACC CCT TTT ACA ATA GCT GCG ACA AAC AAC AAC AAC AAA AAA ACA
Ser Thr Pro Phe Thr Ile Ala Ala Thr Asn Asn Asn Asn Lys Lys Thr

      150                 160                 170
AAA CTT AGG AAT ATA CCT AGC AAA GAA TTC
Lys Leu Arg Asn Ile Pro Ser Lys Glu Phe
```

# Fig.15

```
                10          20          30              40
GAA TTC CCT AGC CTG GGC AAC AGA GTG AGA GTC CGT CTC AAA AAA AAA
Glu Phe Pro Ser Leu Gly Asn Arg Val Arg Val Arg Leu Lys Lys Lys

 50          60          70          80          90
AAA ACA ACA ACA AAA AAA ACA AAC CCA CAA AAC TGC AGC CAC CTA TGT
Lys Thr Thr Thr Lys Lys Thr Asn Pro Gln Asn Cys Ser His Leu Cys

    100         110         120             130
CCC TAC CTC CCC AGC CTC CAG GGC CCC TTC CGG AAT TCC
Pro Tyr Leu Pro Ser Leu Gln Gly Pro Phe Arg Asn Ser
```

# Fig.16

```
           10         20          30          40
GAA TTC GCG GGA ACC CGG GAG GCG GAC GTT GCA GTG AGC CGA GAT CGC
Glu Phe Ala Gly Thr Arg Glu Ala Asp Val Ala Val Ser Arg Asp Arg

 50         60          70          80          90
GCC ACT GCA CTC CAG CCT GGG CGA CAG AGC AAG ACT CTG TCT CAA AAA
Ala Thr Ala Leu Gln Pro Gly Arg Gln Ser Lys Thr Leu Ser Gln Lys

    100         110         120         130
AAA AAA AAC AAA AAC AAA AAG AAG GAC TGG GAG GGT CGG CAG
Lys Lys Asn Lys Asn Lys Lys Lys Asp Trp Glu Gly Arg Gln

 140        150        160        170        180
TAATCGAGGA CCACCTGGCA GTGACAGAGG GTGACCCAGG GCTGGGAGGA

 190        200        210        220        230
TACCCCAGGG GAGACCCCAG GCTCTGAAAA GTGCCTTGCC ATTCAATCTA

 240        250        260        270        280
CTTCAGTAAT AGCATGTGTC ATGGGATAGA TAATAAAATC CGGAGGGGAA

 290        300
AAAATGCTCG CGGAATTC
```

# Fig.17

```
                10          20           30          40
GAA TTC AGC ACA GTT TCC AGA TAC AAG ATT AAT GGA CAC AAA TCA GTA
Glu Phe Ser Thr Val Ser Arg Tyr Lys Ile Asn Gly His Lys Ser Val

 50          60          70          80           90
GCT CTT CCA TAC ATC AAC AGC TAC CAA GCA GAG AAT CAC ATC AAG AAC
Ala Leu Pro Tyr Ile Asn Ser Tyr Gln Ala Glu Asn His Ile Lys Asn

    100         110          120          130          140
TCA ACC CCT TTT ACA ATA GCT GCG ACA AAC AAC AAC AAC AAA AAA ACA
Ser Thr Pro Phe Thr Ile Ala Ala Thr Asn Asn Asn Asn Lys Lys Thr

      150         160          170
AAA CTT AGG AAT ATA CCT AGC AAA GAA TTC
Lys Leu Arg Asn Ile Pro Ser Lys Glu Phe
```

# Fig.18

```
                10              20              30              40
GAA TTC CCT AAA AAA GAA AAA AAA AAA AAA AGA CTT CAG CCA ACA GAT
Glu Phe Pro Lys Lys Glu Lys Lys Lys Lys Arg Leu Gln Pro Thr Asp

 50             60              70              80              90
CAG AAC GCA GAA AAT GCA TTT GCC TCA GTA GTG AGT CGG CAG AAT TC
Gln Asn Ala Glu Asn Ala Phe Ala Ser Val Val Ser Arg Gln Asn
```

# Fig.19

```
           10          20          30          40
GAA TTC TTC ACG GAA TTG GAT GGG GTG CGG CTA CAC AGG TAC GCT CCG
Glu Phe Phe Thr Glu Leu Asp Gly Val Arg Leu His Arg Tyr Ala Pro

 50          60          70          80          90
GCG TGC AAG CCT CTC CTA CGG GAT GAG GTC ACA TTC CAG GTC GGG CTC
Ala Cys Lys Pro Leu Leu Arg Asp Glu Val Thr Phe Gln Val Gly Leu

    100         110         120         130         140
AAC CAA TTC CCG GTT GGG TCA CAG CTC CCA TGT GAA CCC GAA CCG GAT
Asn Gln Phe Pro Val Gly Ser Gln Leu Pro Cys Glu Pro Glu Pro Asp

     150         160         170         180         190
GTA ATG GTG GTC ACC TCT ATG CTC ACC GAC CCC TCC CAT ATT ACA GCA
Val Met Val Val Thr Ser Met Leu Thr Asp Pro Ser His Ile Thr Ala

        200         210         220         230         240
GAG ACG GCT AAG CGT AGG CTG GCC AGA GGG TCT CCC CCT TCT TTG GCC
Glu Thr Ala Lys Arg Arg Leu Ala Arg Gly Ser Pro Pro Ser Leu Ala

           250         260         270         280
AGC TCT TCA GCT AGT CAG TTG TCT GCG CCC TCC TTG AAG GCG ACA TGC
Ser Ser Ser Ala Ser Gln Leu Ser Ala Pro Ser Leu Lys Ala Thr Cys

 290         300         310         320         330
ACC ACC CGT CAT GAC TCC CCG GAC GCT GAC CTC ATA GAG GCC AAC CTC
Thr Thr Arg His Asp Ser Pro Asp Ala Asp Leu Ile Glu Ala Asn Leu

    340         350         360         370         380
CTG TGG CGG CAG GAG ATG GGC GGG AAC ATC ACC CGT GTG GAG TCA GAG
Leu Trp Arg Gln Glu Met Gly Gly Asn Ile Thr Arg Val Glu Ser Glu

     390         400         410         420         430
AAT AAG GTA GTG ATT TTG GAC TCT TTT GAA CCG CTT CGG GTG GAG GAG
Asn Lys Val Val Ile Leu Asp Ser Phe Glu Pro Leu Arg Val Glu Glu

        440         450         460         470         480
GAT GAG AGG GAA GTA TCC GTA GCG GCG GAT TTC AGT GAC TTG AAT GCA
Asp Glu Arg Glu Val Ser Val Ala Ala Asp Phe Ser Asp Leu Asn Ala

          490        500        510        520        530
GAA TGAATCCCGT GGCTCACTTC CTAGACTATT TGCCAAAGAA GATGTTGCCC
Glu

     540        550        560        570        580
TGGCCATGAT CAAGATGACA CAAACGGTGG CCTTTTGCAG GGAGAACCGC

     590        600        610        620        630
CGTGGAGGCC TGTGTCTGTG GCACTGGTAG CTTCTCTCTG CAGGCAAAGA

     640        650        660
CCCCATGGCT TAGTTCTTCA TCAGAGTGAG AATTC
```

# Fig.20

```
                10           20            30            40
GAA TTC TTC ACG GAG TTG GAT GGG GTA CGG CTG CAC AGG TAC GCT CCG
Glu Phe Phe Thr Glu Leu Asp Gly Val Arg Leu His Arg Tyr Ala Pro

 50             60            70           80            90
GCG TGC AAG CCA CTC CTA CGG GAT GAG GTC ACA TTC CAG GTC GGG CTC
Ala Cys Lys Pro Leu Leu Arg Asp Glu Val Thr Phe Gln Val Gly Leu

     100          110           120           130          140
AAC CAA TTT CCA GTT GGA TCA CAG CTC CCA TGT GAG CCC GAG CCG GAT
Asn Gln Phe Pro Val Gly Ser Gln Leu Pro Cys Glu Pro Glu Pro Asp

       150           160           170          180          190
GTA GCG GTG CTC ACT TCC ATG CTC ACC GAC CCC TCC CAC ATT ACA GCA
Val Ala Val Leu Thr Ser Met Leu Thr Asp Pro Ser His Ile Thr Ala

          200          210           220          230          240
GAG ACG GCT AAG CGT AGG CTG GCC AGG GGG TCC CCC CCC TCC TTG GCC
Glu Thr Ala Lys Arg Arg Leu Ala Arg Gly Ser Pro Pro Ser Leu Ala

                250          260          270          280
AGC TCT TCA GCT AGT CAG TTG TCT GCG CCC TCC TTG AAG GCG ACA TGC
Ser Ser Ser Ala Ser Gln Leu Ser Ala Pro Ser Leu Lys Ala Thr Cys

 290           300           310          320          330
ACT ACC CAC CAT GAC TCC CCG GAC GCT GAC CTC ATC GAG GCC AAC CTC
Thr Thr His His Asp Ser Pro Asp Ala Asp Leu Ile Glu Ala Asn Leu

     340          350           360           370          380
CTG TGG CGG CAG GAG ATG GGA GGA AAC ATC ACC CGC GTG GAG TCA GAG
Leu Trp Arg Gln Glu Met Gly Gly Asn Ile Thr Arg Val Glu Ser Glu

       390           400           410          420          430
AAT AAG GTA GTA ATT CTA GAC TCT TTT GAC CCG CTC CGA GCG GAG GAG
Asn Lys Val Val Ile Leu Asp Ser Phe Asp Pro Leu Arg Ala Glu Glu

          440          450           460          470
GAT GAG AGG GAA GTG TCC GTT GCG GCG GAG ATC CTG CGG AAG ACC AG
Asp Glu Arg Glu Val Ser Val Ala Ala Glu Ile Leu Arg Lys Thr
```

Fig.21

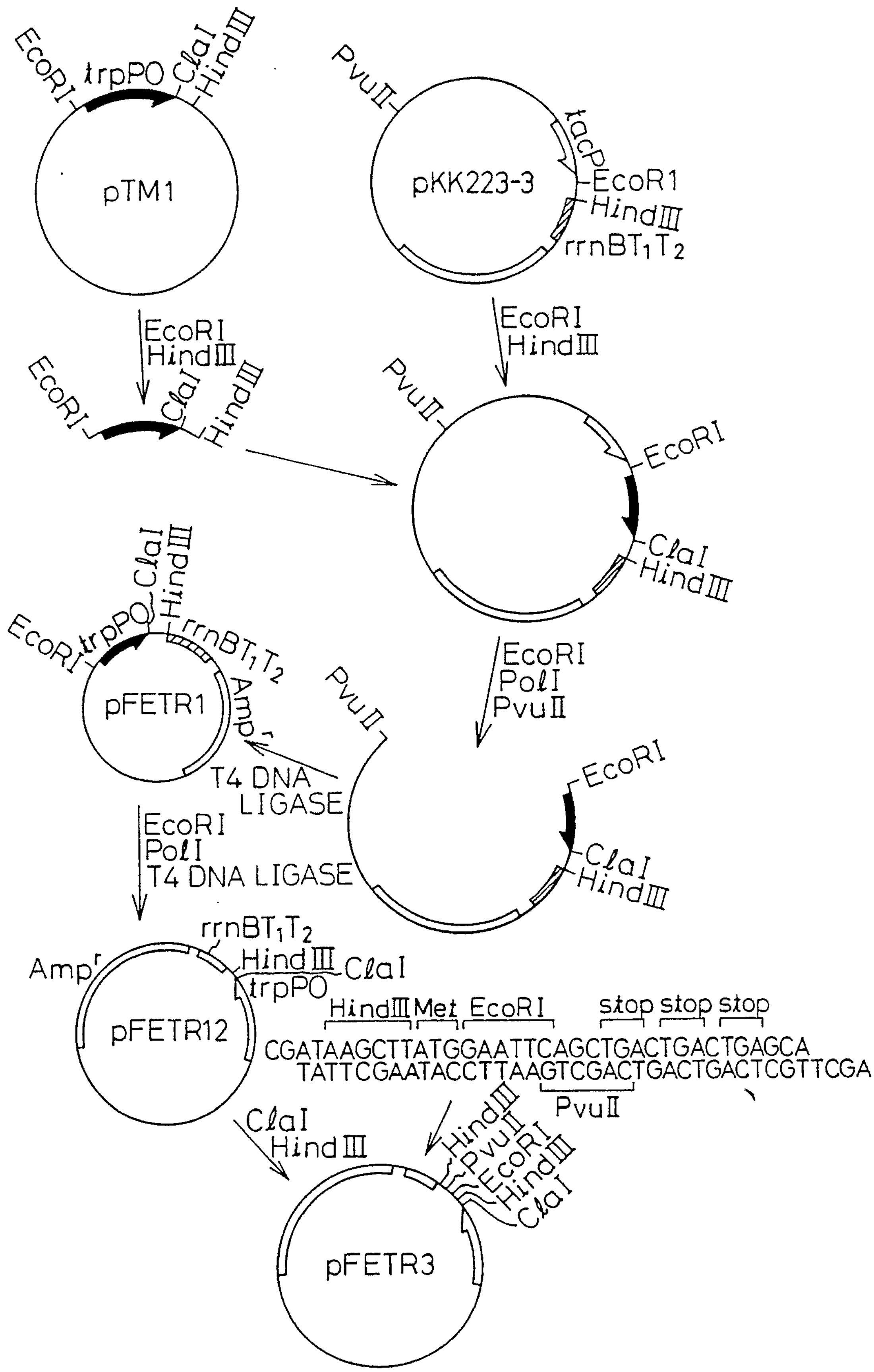
EcoRI
trpPO
ClaI
HindIII
pTM1
PvuII
tacP
EcoR1
HindIII
rrnBT1T2
pKK223-3
EcoRI
HindIII
EcoRI
PolI
PvuII
pFETR1
Ampr
T4 DNA
LIGASE
EcoRI
PolI
T4 DNA LIGASE
pFETR12
HindIII Met EcoRI
stop stop stop
CGATAAGCTTATGGAATTCAGCTGACTGACTGAGCA
TATTCGAATACCTTAAGTCGACTGACTGACTCGTTCGA
PvuII
pFETR3

Fig.22

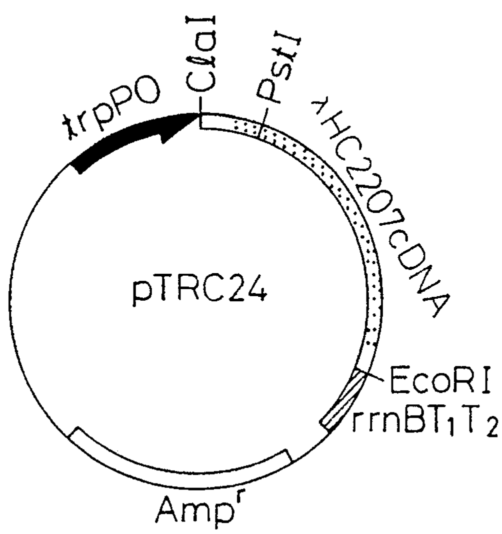
trpPO
ClaI
PstI
λHC2207cDNA
pTRC24
EcoRI
rrnBT1T2
Amp^r

Fig.23

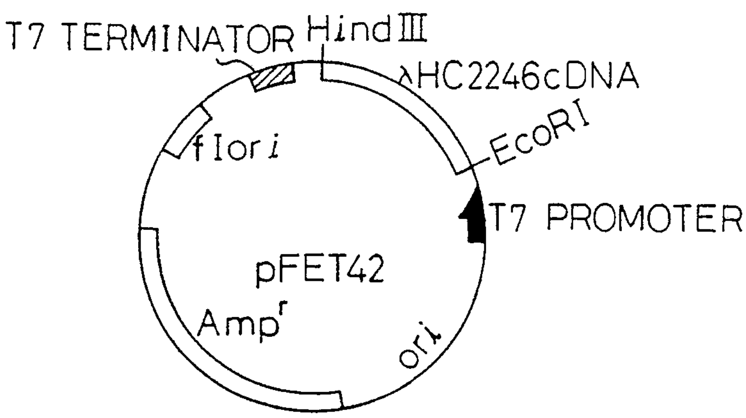
T7 TERMINATOR
HindIII
λHC2246cDNA
EcoRI
f1ori
T7 PROMOTER
pFET42
Amp^r
ori